# EUROPEAN PATENT SPECIFICATION

(11) **EP 1 752 771 B1**
(45) Date of publication and mention of the grant of the patent: **04.03.2009**
(21) Application number: 06254200.6
(22) Date of filing: 09.08.2006
(51) Int. Cl.: G01N 33/68

(54) **Biomolecule specificity detection and selection method and apparatus**
Detektion der Biomolekül Spezifität sowie Selektionsmethode und Apparat
Détection et la spécificité d'une biomolécule, méthode de sélection et appareil

(30) Priority: 09.08.2005 US 199243
(43) Date of publication of application: 14.02.2007
(73) Proprietor: Genetix Limited, New Milton, Hampshire BH25 5NN (GB)
(72) Inventor: Burke, Julian Francis, New Milton, Hampshire BH25 5NN (GB); Mann, Christopher, New Milton, Hampshire BH25 5NN (GB)
(74) Representative: Harding, Charles Thomas

(56) References cited:
- EP-A- 0 293 649
- EP-A1- 1 502 649
- WO-A-20/05020924
- COLIGAN ET AL: 'current protocols in immunology' CURRENT PROTOCOLS IN IMMUNOLOGY vol. 1, 01 January 1991, pages 2.1.1 - 2.1.22, XP003001177

## Description

### FIELD

This invention relates to the field of molecular and cell biology, in particular to the field of detection of biological entities such as biomolecules, and their specificity.

### BACKGROUND

Many molecular biology techniques depend on cloning individual cells from a mixture of cells.

For example, in the production of monoclonal antibodies, an essential step is hybridoma selection, including the separation and culture of individual hybridoma clones (fused myelomas and primary mouse cells). After cell fusion, the traditional way of selecting for monoclonality is to plate out single cells into 96-well dishes. This is repeated until clonality is assured.

Similarly, understanding gene function and identification of pharmaceutical leads requires the establishment of cell lines containing transfected genes expressed at an appropriate level. Standard techniques require the co-transfection of a gene with a dominant selectable marker followed by selection for growth for example in an antibiotic such as G418 or hygromycin. The resulting colonies are then picked by hand and further analysed for gene expression (RT-PCR) and functional expression.

Ascertaining optimal conditions for stem cell growth and differentiation requires broad testing of growth factors and culture conditions. The evaluation of a particular treatment requires a statistical approach on a large number of individual cells. One way to achieve this is to use numerous culture dishes, several for each treatment.

This process of cloning out may be modified and automated through the use of robots. Thus, for example, the ClonePix robot (manufactured by Genetix) implements this process by picking individual colonies directly from standard semi-solid media, the media preventing migration of the dividing cells. Thus, an imaging head captures images of colonies growing in the medium under white light, and software routines allow the separation and detection of individual colonies. A picking head then picks individual colonies into a 96-well plate.

One advantage of a robot implemented picking method is that colonies grown for as few as 3 days can be picked into 96-well plates. The picking speed can be up to 400 clones per hour and graphic software allows the user to select colonies on the basis of size, shape, brightness and proximity. Furthermore, the software allows stratification of clones into slow, medium and fast growing cells, and clones of the same class may be grouped in the same 96-well plate. This gives rise to considerable savings in subsequent tissue culture steps as all wells can be processed at the same time.

However, the robot implemented cloning method relies on visualisation solely of colony size. Thus, the image capture only provides information on the size of the colony, and all colonies within a certain size range are picked. It is known for example that different hybridoma clones are capable of producing varying amounts of antibody. No information is provided or processed as to the productivity of different cells (i.e., the quantity of product produced or secreted), and the robot implemented cloning method therefore cannot discriminate between a high-producing hybridoma cell or colony and a low-producing hybridoma cell or colony. With regard to transfected cells, the robot cannot distinguish between clones with different levels of expression and/or secretion of recombinant protein.

A further disadvantage of the robot implemented cloning method described is that it is not capable of distinguishing at a molecular level the different polypeptides secreted by the various clones that are growing in the medium. Thus, in the case of hybridoma selection, no information is provided on the class, specificity, affinity or avidity of the antibody that is secreted by a particular cell or colony. Where stem cells are being cloned out, the robot implemented cloning method cannot distinguish between different types of stem cells (i.e., markers present on the cell surface).

WO 2005/020294 describes a method to obtain high expression clones of mammalian cells. The method involves contacting cells with a capture molecule that interacts with the polypeptide of interest such that the interaction indicates relative expression of the polypeptide for each cell or group of cells. The capture molecule is required to be capable of binding or reacting with the polypeptide of interest.

EP 0 293 649 describes a method of selecting hybridomas which secrete antibodies against creatine kinase-MM. Such hybridomas are detected by exposing them to rabbit anti-rat-Fab antibodies.

Neither WO 2005/020294 or EP 0 293 649 describes a method of distinguishing between cells or colonies producing different polypeptides or selecting cells or colonies based on the nature of these polypeptides.

It is a general object of the invention to provide a method of distinguishing colonies based on the nature of the product produced or secreted by that colony, in particular the quantity of the product and its binding ability.

### SUMMARY

According to a 1^{st} aspect of the present invention, we provide a method of detecting a polypeptide of interest produced by a cell or a cell colony, the method comprising exposing the polypeptide to a class marker capable of identifying a class of polypeptides to which the polypeptide of interest belongs and a specificity marker capable of identifying a specific binding ability of the polypeptide of interest and detecting binding of the polypeptide to the class marker and the specificity marker.

Preferably, the class marker comprises a polyclonal antibody capable of binding to polypeptides of a class to which the polypeptide of interest belongs.

Preferably, the specificity marker comprises a binding partner capable of specifically binding to the polypeptide of interest.

Preferably, the cell or cell colony is disposed on or in solid or semi-solid media, preferably methylcellulose media.

Preferably, the polypeptide is secreted by the cell.

Preferably, binding between the class marker and the polypeptide is detected to provide a measure of the amount or presence of the polypeptide of interest.

Preferably, the class marker-polypeptide complex forms a halo or aura around the cell or cell colony.

Preferably, the size or extent of the halo is detected to provide a measure of the amount of polypeptide produced by the cell or cell colony.

Preferably, binding between the specificity marker and the polypeptide is detected to provide a measure of the binding specificity of the polypeptide of interest.

Preferably, binding between the polypeptide and the class marker and binding between the polypeptide and the specificity marker is detected simultaneously.

Preferably, the class marker comprises a small molecule, preferably a synthetic small molecule.

Preferably, the class marker or the specificity marker or both is labelled with a reporter, preferably a fluorescent molecule, preferably a fluorophore, more preferably a fluorochrome.

Preferably, the class marker and the specificity marker are labelled with two different fluorophores emitting fluorescent light of different wavelengths.

Preferably, the fluorophores comprise fluorescein, tetramethylrhodamine or phycoerythrin.

Preferably, the polypeptide of interest comprises a recombinant polypeptide, preferably expressed by a transfected cell such as a suspension adapted cell-line.

Preferably, the cell or cell colony is a hybridoma cell or cell colony, including a transfected hybridoma, and the polypeptide of interest comprises an immunoglobulin or antibody.

Preferably, the class of polypeptides is an Ig class, preferably an IgG class.

Preferably, the class marker comprises a polyclonal anti-IgG antibody.

Preferably, the specificity marker comprises an antigen or epitope of the immunoglobulin or antibody.

Preferably, the polypeptide of interest comprises a receptor, preferably a cell surface receptor.

Preferably, the class of polypeptides is a receptor subclass, preferably a seven transmembrane (7TM) receptor subclass.

Preferably, the class marker comprises an antibody capable of binding to a receptor subclass, preferably an antibody capable of binding to a seven transmembrane (7TM) receptor subclass.

Preferably, the specificity marker comprises a ligand, preferably an affinity ligand, of the receptor.

There is provided, according to a 2^{nd} aspect of the present invention, a method of identifying the cell type of a cell or colony, the method comprising detecting whether the cell or colony produces a polypeptide known to be associated with a particular cell type by a method according to the 1^{st} aspect of the invention.

We provide, according to a 3^{rd} aspect of the present invention, a method of selecting a cell or colony of a cell type of interest from a plurality of cells or colonies, the method comprising detecting whether a candidate cell or colony produces a polypeptide known to be associated with the cell type of interest by a method according to the 1^{st} or 2^{nd} aspect of the invention.

As a 4^{th} aspect of the present invention, there is provided a method according to the 1^{st}, 2^{nd} or 3^{rd} aspect of the invention for assessing the productivity of a hybridoma cell or cell colony in producing an antibody of interest, and optionally, the specificity of said antibody.

We provide, according to a 5^{th} aspect of the present invention, a method of selecting a productive cell or cell colony from a plurality of cells or cell colonies, the method comprising: (a) assessing the amount of polypeptide of interest produced by a relevant cell or cell colony by a method according to the 1^{st} aspect of the invention; (b) comparing said amount to a predetermined amount; (c) selecting the cell or cell colony if it meets or exceeds the predetermined amount.

Preferably, the method is used for selecting a set of productive cells or cell colonies which meet or exceed the predetermined amount.

Preferably, the method detects a specificity of a polypeptide produced by the cell or cell colony and further comprises the steps of: (a) assessing the binding specificity of polypeptide of interest produced by the cell or cell colony by a method according to the 1^{st} aspect of the invention; (b) selecting the cell or cell colony if it produces a polypeptide which binds specifically to a binding partner of interest.

Preferably, the plurality of cells or cell colonies is disposed on a planar substrate such as a culture dish or plate.

The present invention, in a 6^{th} aspect, provides a method of picking a cell or cell colony productive for a polypeptide, the method comprising assessing the amount of polypeptide of interest produced by the cell or cell colony by method according to the 1^{st} aspect of the invention, and picking that cell or cell colony.

As a 7^{th} aspect of the invention, we provide for use of a robot equipped with a head for manipulating biological samples, the use comprising: providing a sample container containing at least one cell capable of producing a polypeptide of interest, wherein the polypeptide has been exposed: (a) to a class marker capable of identifying a class of polypeptides to which the polypeptide of interest belongs; and (b) to a specificity marker capable of identifying a specific binding ability of the polypeptide of interest; arranging the sample container at an imaging station; making a spectroscopic measurement by illuminating the at least one cell from below and collecting light from the at least one cell also from below in order to detect binding of the polypeptide to the class marker or the specificity marker or both; and manipulating the at least one cell with the head based on the spectroscopic measurement of the binding of the polypeptide.

The practice of the present invention will employ, unless otherwise indicated, conventional techniques of chemistry, molecular biology, microbiology, recombinant DNA and immunology, which are within the capabilities of a person of ordinary skill in the art. Such techniques are explained in the literature. See, for example, J. Sambrook, E. F. Fritsch, and T. Maniatis, 1989, Molecular Cloning: A Laboratory Manual, Second Edition, Books 1-3, Cold Spring Harbor Laboratory Press; Ausubel, F. M. et al. (1995 and periodic supplements; Current Protocols in Molecular Biology, ch. 9, 13, and 16, John Wiley & Sons, New York, N.Y.); B. Roe, J. Crabtree, and A. Kahn, 1996, DNA Isolation and Sequencing: Essential Techniques, John Wiley & Sons; J. M. Polak and James O'D. McGee, 1990, In Situ Hybridization: Principles and Practice; Oxford University Press; M. J. Gait (Editor), 1984, Oligonucleotide Synthesis: A Practical Approach, Irl Press; D. M. J. Lilley and J. E. Dahlberg, 1992, Methods of Enzymology: DNA Structure Part A: Synthesis and Physical Analysis of DNA Methods in Enzymology, Academic Press; Using Antibodies : A Laboratory Manual : Portable Protocol NO. I by Edward Harlow, David Lane, Ed Harlow (1999, Cold Spring Harbor Laboratory Press, ISBN 0-87969-544-7); Antibodies : A Laboratory Manual by Ed Harlow (Editor), David Lane (Editor) (1988, Cold Spring Harbor Laboratory Press, ISBN 0-87969-314-2), 1855, Lars-Inge Larsson "Immunocytochemistry: Theory and Practice", CRC Press inc., Baca Raton, Florida, 1988, ISBN 0-8493-6078-1, John D. Pound (ed); *"*Immunochemical Protocols, vol 80", in the series: "Methods in Molecular Biology", Humana Press, Totowa, New Jersey, 1998, ISBN 0-89603-493-3, Handbook of Drug Screening, edited by Ramakrishna Seethala, Prabhavathi B. Fernandes (2001, New York, NY, Marcel Dekker, ISBN 0-8247-0562-9); Lab Ref: A Handbook of Recipes, Reagents, and Other Reference Tools for Use at the Brench, Edited Jane Roskams and Linda Rodgers, 2002, Cold Spring Harbor Laboratory, ISBN 0-87969-630-3; and The Merck Manual of Diagnosis and Therapy (17th Edition, Beers, M. H., and Berkow, R, Eds, ISBN: 0911910107, John Wiley & Sons).

### BRIEF DESCRIPTION OF THE FIGURES

Figure 1 is a photomicrograph showing fluorescence detection of specific antibody-secreting mammalian cells using fluorescence tagged antigen, under white light imaging.
Figure 2 is a photomicrograph showing fluorescence detection of specific antibody-secreting mammalian cells using fluorescence tagged antigen, under fluorescence imaging.
Figure 3 is a photomicrograph of colonies from the experiment depicted in Figures 1 and 2 at higher resolution, under white light imaging.
Figure 4 is a photomicrograph of colonies from the experiment depicted in Figures 1 and 2 at higher resolution, under fluorescence imaging.
Figure 5 is a photomicrograph of a different field showing other colonies from the experiment depicted in Figures 1 to 4 at maximum resolution, under white light imaging.
Figure 6 is a photomicrograph of a different field showing other colonies from the experiment depicted in Figures 1 to 4 at maximum resolution, under fluorescence imaging.
Figure 7 is a photomicrograph of colonies from the experiment depicted in Figures 1 to 6 showing actual detail at maximum resolution, under white light imaging.
Figure 8 is a photomicrograph of colonies from the experiment depicted in Figures 1 to 6 showing actual detail at maximum resolution, under fluorescence imaging.
Figure 9 is a photomicrograph of a negative control for the experiment depicted in Figures 1 to 8 under white light imaging (presence of FITC tagged antigen (~60 kDa) but in the absence of anti-IgG antibody).
Figure 10 is a photomicrograph of a negative control for the experiment depicted in Figures 1 to 8 under fluorescence imaging (presence of FITC tagged antigen (~60 kDa) but in the absence of anti-IgG antibody).
Figure 11 is a photomicrograph of a negative control for the experiment depicted in Figures 1 to 8 under white light imaging (presence of untagged anti-IgG antibody and of FITC tagged control antigen).
Figure 12 is a photomicrograph of a negative control for the experiment depicted in Figures 1 to 8 under fluorescence imaging (presence of untagged anti-IgG antibody and of FITC tagged control antigen).

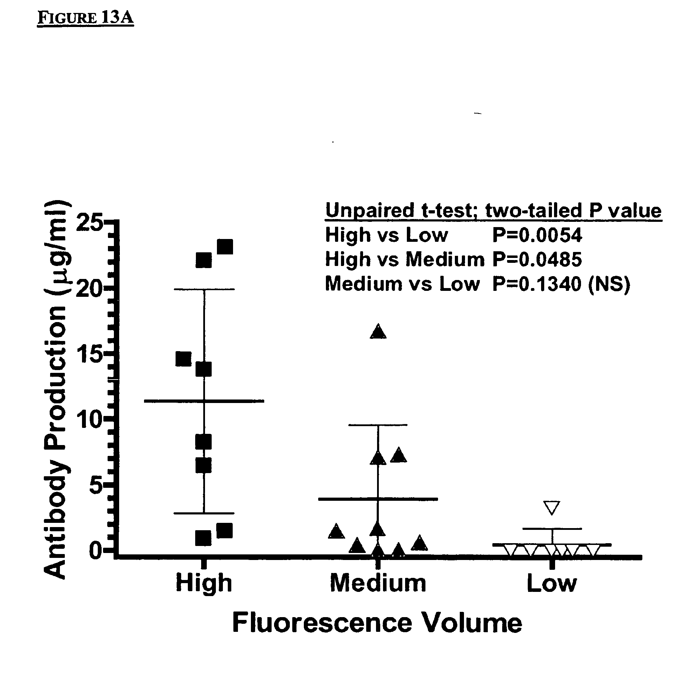

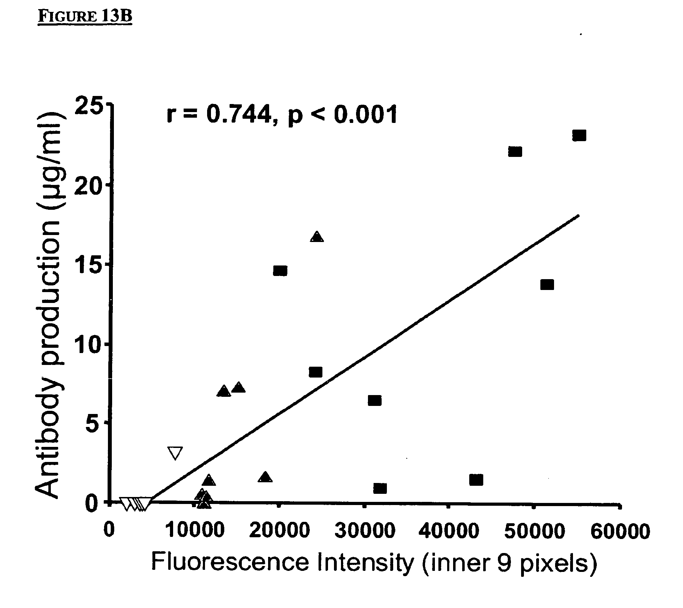

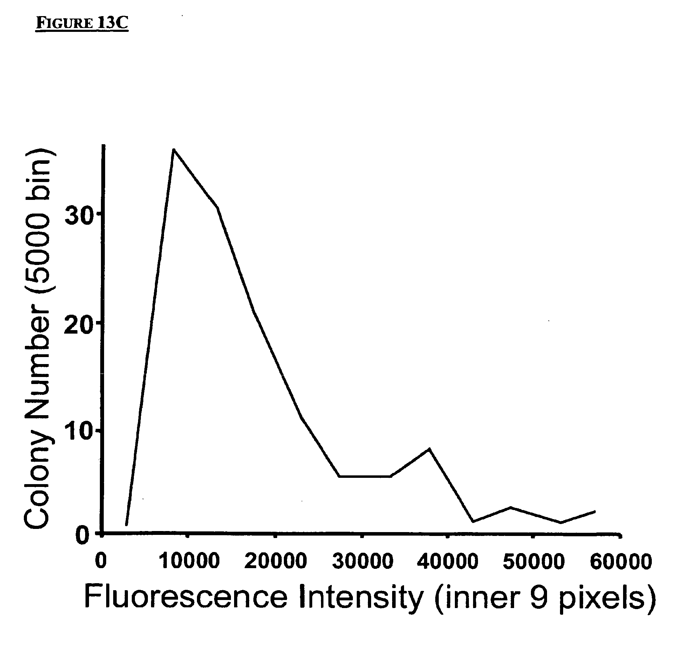

Figure 13: Relationship between fluorescence detection and antibody productivity. IgG1 expressing NS0 cells were grown into colonies for 7 days in semi-solid medium, and quantified for antibody production using FITC anti human IgG. High, medium and low fluorescing colonies were picked to a 96-Well plate, grown on for 7days and transferred to 24-Well plates for 11 days until <30% viable. Supernates were collected and antibody production measured using a Protein A / HPLC capture and quantitation method. A: Comparison of production in the three groups. B: Correlation of production with colony fluorescence intensity. C: Population distribution of all colonies in the original sample (n=123) from which the high, medium and low fluorescing colonies were selected.
Figure 13A is a graph plotting fluorescence volume against antibody production (µg/ml)
Figure 13B is a graph plotting fluorescence intensity of inner 9 pixels against antibody production (µg/ml).
Figure 13C is a graph plotting fluorescence intensity of inner 9 pixels against colony number (5000 bin).
Figure 14 is an image of a halo.
Figure 15 shows the image of Figure 14 after processing by the image processing software, as used for detection and measurement of the halo.
Figure 16 is a perspective view of a robotic apparatus for carrying out methods according to the invention.
Figure 17 is a schematic sectional side view showing the sample excitation and collection paths in the vicinity of the sample using a well plate as an example sample container.
Figure 18 is a schematic plan view of the sample vicinity with a well plate as the sample container.
Figures 19A, 19B and 19C are perspective and orthogonal side views of the optics sub-assembly arranged below the main bed of the apparatus of Figure 16.
Figure 20 is a block schematic diagram showing the control system of the apparatus.
Figure 21 is an image taken on ClonePixF1 showing two Hybridoma colonies grown for 7 days in methyl cellulose based semi-solid medium. The two colonies, which differ in size, have been circled in red to distinguish them from the background.
Figure 22 is an image taken on ClonePixFl in the AF546 fluorescent wavelength. The same two colonies as in Figure 1 have been circled. A signal in this wavelength is indicative of expression of an antibody of the IgG class.
Figure 23 is an image taken on ClonePixF1 in the FITC fluorescent wavelength. The same two colonies as in Figure 1 and 2 have been circled. A signal in this wavelength is indicative of specificity to the antigen.

### DETAILED DESCRIPTION

We describe methods of detecting a polypeptide of interest which is produced by a cell or colony. The determination as to whether a cell or colony produces or does not produce the polypeptide of interest may be used as a means to determine whether the colony or cell is one of interest. Colonies or cells of interest may then be selected and picked for further study.

The detection method relies on the use of one or more binding agents or markers which are chosen to match the nature or characteristics of the polypeptide of interest.

According to the methods described here, a candidate polypeptide is exposed to two or more markers simultaneously or in sequence. A first marker, in this document referred to as the "class marker", identifies the general nature or class of the polypeptide produced by a particular cell or colony such as the presence of a common motif shared by the class. Specifically, the class marker preferably only binds to members of that class.

A second marker, in this document referred to as the "specificity marker" identifies a specific nature of that polypeptide such as a binding ability. Polypeptides which have that binding ability are bound to the specificity marker. The "specific nature" will be chosen by the skilled person based on his knowledge of the properties of the polypeptide of interest. At the very least, one specificity marker is employed in the method described. However, and as described in further detail below, more than one specificity marker may be used.

The use of the first class marker establishes a sub-selection of polypeptides, that is to say, only those of the particular class of interest. Accordingly, the first class marker can be considered to eliminate from consideration polypeptides not of the class to which the polypeptide of interest belongs. The use of the second specificity marker narrows down that sub-selection only to those which fulfil the required specific nature chosen, e.g., binding ability. Further specificity markers may be used to further narrow down the choice.

Accordingly, we provide for detection by identifying polypeptides which bind to both the first class marker and the one or more second specificity marker. Advantageously, at least the specificity marker is labelled with a reporter which is capable of emitting a signal in order to ease detection. Colonies or cells which emit signal may be chosen and picked for further manipulation.

In preferred embodiments, any of the steps set out in relation to the detection method, such as exposing the polypeptide to a marker, detection of binding, as well as associated steps such as selection and/or picking of cells or colonies of interest may be conducted using automated robotic apparatus. In preferred embodiments, the robotic apparatus comprises a ClonePix FL apparatus (Genetix, New Milton, United Kingdom).

In preferred embodiments, the polypeptide is secreted from a cell or colony of cells which is grown on the surface of or within solid or semi-solid media. Thus, preferably, the cell or colony may be grown on agar, agarose, or methylcellulose media. In preferred embodiments, the cells or colonies are grown on a Petri dish or other similar container, although it will be appreciated that other containers may also be used, such as well plates, particularly 1 well plates, 4 well plates, 6 well plates, microtitre dishes, etc.

The cell or colony of cells may comprise any cultured cell or cell line, as known in the art. Included are prokaryotic cells and eukaryotic cells, including bacteria, yeast, insect and mammalian cells. A list of known cell lines is set out in the Cell Line Data Base (Istituto Nazionale per la Ricerca sul Cancro, Genova, Italy) and the ECACC European Collection of Cell Cultures. Specific examples of cells include E. *coli* cells, CHO cells, HeLa cells, African green monkey cells, Sf9 cells, etc. Such cells may be transfected with suitable expression vectors to enable expression of polypeptides, as described in further detail below. Other cells particularly suitable for use in the methods described here are fused cell lines, including hybridoma cell lines.

It will be evident that the class marker and the specificity marker(s) can be provided as separate molecules, such as two separate molecules where only one specificity marker is used, or as a single molecule. The single molecule may comprise a conjugate, fusion, etc of a class marker or specificity marker or markers, as described in further detail below.

Moreover, where the class marker and the specificity marker(s) are applied as separate molecules, the markers may be applied to the polypeptide simultaneously or in sequence, in any order. For example, the specificity marker(s) may be applied to the polypeptide followed by the class marker. However, the preferred order is to apply the class marker to the polypeptide followed by the specificity marker or markers.

Preferably, the class marker is applied and an interval allowed to elapse to enable it to bind to the polypeptide, before the specificity marker or markers is applied. Preferably, the interval is long enough for the formation of a halo or aura, described below. One or more manipulations may be carried out in the interval. For example, a detection step to visualise the bound class marker, preferably in the form of a halo, may be carried out prior to the application of the specificity marker.

The extent of the halo or fluorescence may be assessed to provide an indication of the amount of polypeptide produced by the particular cell or colony; in this manner, productive (high producing) colonies may be chosen for further study.

Specifically, the method described herein enables colonies or cells of interest, e.g., which produce polypeptides of interest detectable by the class marker and the specificity marker, to be identified. Advantageously, the colonies are visualised and imaged, and identified by software according to whether or not they emit a signal. Other characteristics, such as size, may also be used for identifying relevant colonies or cells. Selected colonies or cells may then be picked and replated, for example into 96 well plates, for growing on, using for example a ClonePix FL robotic apparatus (Genetix, New Milton, United Kingdom).

The class marker and the specificity marker(s) may be chosen based on the knowledge of the nature or characteristics of the polypeptide of interest, as described in further detail below.

The invention enables the selection of a polypeptide of interest, and hence a cell or colony which produces it, by allowing the selection of polypeptides which bind both the class marker and the specificity marker, and which therefore fulfil both criteria. Other criteria may be applied. For example, a cell or colony may be chosen on the basis of its productivity, i.e., how much polypeptide it produces. Thus, the methods described here enable the selection of high producing hybridoma colonies, for example.

The method may be applied to a number of cells or colonies, preferably a plurality of cells or colonies, simultaneously, and is capable of detecting only those cells or colonies which produce the particular product of interest. Our method is therefore able to discriminate among a plurality of polypeptides, which may be heterogenous in sequence or concentration (as a result, for example, of different expression efficiencies of the cells or colonies in question), to allow the detection and selection of a relevant cell or colony (among other cells or colonies) which produces a particular polypeptide of interest.

The method disclosed is capable of detecting a polypeptide of interest produced by a cell or a cell colony. The polypeptide is generally one which is expressed by the cell or colony, including polypeptides which are secreted by the cell or cell colony, for example into the medium in which they are growing. Also included are those polypeptides which are produced by the cell but retain some association therewith, for example by being presented on the cell surface, or retained for example within a cell wall or a periplasmic space.

### USES OF DETECTION METHOD

The detection methods described here enable the identification of the cell type of a cell or colony. The method comprises comprising detecting whether a particular cell or colony produces a polypeptide known to be associated with a particular cell type. The associated protein may be defined by a class or binding specificity, and the choice of class and specificity marker will depend on the nature of the associated polypeptide.

The methods also allow the selection of a cell or colony of a cell type of interest from a plurality of cells or colonies. The cell or colony of interest is defined by the ability to produce a polypeptide known to be associated with the cell type of interest, which polypeptide is detected by the detection method set forth in this document. The colony or cell of interest may then be optionally picked for further study or manipulation, for example into a 96 well plate. Advantageously, the selection, including visualisation of colonies, or picking or both is conducted using a robotic apparatus such as a ClonePix FL apparatus (Genetix, New Milton, United Kingdom).

The detection method may be used for picking a cell or cell colony productive for a polypeptide, by assessing the amount of polypeptide of interest produced by the cell or cell colony and picking that cell or cell colony. It will also be clear that similarly the detection method may be adapted to assess the productivity of a cell or colony in producing a protein of interest, for example a hybridoma cell or cell colony in producing an antibody of interest. The productivity which is determined may be compared to a predetermined cut off point, and only those cells or colonies which meet or exceed that target may be chosen. Thus, the detection method may be employed in a method of selecting a productive cell or cell colony from a plurality of cells or cell colonies.

The detection method may also be adapted to detect a specificity of a polypeptide produced by the cell or cell colony. Thus, such a method may include a step of assessing the binding specificity of polypeptide of interest produced by the cell or cell colony by a use of the detection method, and selecting the cell or cell colony if it produces a polypeptide which binds specifically to a binding partner of interest.

We also provide for a method of selecting a colony of cells which secretes a polypeptide of interest, in which the colony is grown on semi-solid media; the method comprising: (a) forming a halo comprising an aggregate of secreted polypeptide around the cell or cell colony; (b) detecting binding between secreted polypeptide and a specificity marker capable of identifying a specific binding ability of the polypeptide of interest; and (c) selecting a colony based on halo formation and binding of the specificity marker.

It will be evident that the methods described here enable detection of polypeptides produced by cells. In specific embodiments, the cells are intact, and preferably in the form of a colony. However, it will be evident to the skilled person that the cells need not necessarily be intact in order for the polypeptide to be detected. For example, viral infection of a cell may cause it to produce virally encoded polypeptides, and these polypeptides are suitable for detection using the methods described here. The association of the expressed polypeptide with the viral carrier may be used as a means to pick both the cell harbouring the virus, as well as the virus itself.

Thus, for example, the detection method described here may be employed in expression cloning; viz, a bacteriophage lambda gt11 library may be used to infect *E. coli* cells, which are plated out onto agar medium. The *E*. *coli* cells form a lawn on the surface of the plate, and infected cells are lysed, producing a plaque (a clear portion of the lawn). The expressed virally encoded polypeptide may be detected using a suitable class marker and specificity marker, and the plaque of interest picked for further subcloning if desired. A similar system may be employed for expression cloning using baculovirus infection of Sf9 insect cells, or an equivalent mammalian cell system.

### CLASS MARKER

The method comprises the use of a class marker, which may also be regarded as a quantity marker. The class marker is capable of revealing the presence of at least some, preferably substantially all, members of a class of polypeptides to which the polypeptide of interest belongs.

For this purpose, the class marker preferably comprises a binding agent capable of binding to members of that class. Preferably, the class marker comprises a binding agent capable of binding to most or substantially all members of that class. The class marker may comprise any molecule, so long as it has affinity, preferably specificity, for members of that class. For example, where the class of polypeptide comprises immunoglobulins, the class marker may comprise Protein A or Protein G. Protein A is a surface protein of *Staphylococcus aureus* which binds to IgG molecules via their Fc regions.

The class marker comprises a small molecule, by which term we mean a molecule which has a molecular weight below 50 kDa, more preferably below 25 kDa, more preferably below 10 kDa, more preferably below 1 kDa, more preferably below 500 Da, most preferably below 25 Da. Preferably, the small molecule comprises an organic molecule. The small molecule may comprise any of the known biomolecules which exist in organisms, such as hormones, peptides, amino acids, nucleic acids, etc. The class marker may be synthetic or natural. In highly preferred embodiments, the class marker comprises a synthetic small molecule.

The class marker may preferably comprise an antibody or immunoglobulin with the relevant binding ability. The class marker comprises an antibody which is capable of specifically binding to a conserved domain or epitope which is shared by members of the class of polypeptides to which the polypeptide of interest belongs. Specific examples are provided below.

The class marker may comprise a polyclonal antibody or a monoclonal antibody, which may be engineered. In highly preferred embodiments, the class marker comprises a polyclonal antibody, such as a goat or rabbit anti-polypeptide antibody raised for example against a mouse or human IgG. The antibody may be whole, or it may comprise a fragment thereof, preferably a binding fragment such as a F(ab')₂ fragment. Where the term antibody is used in this document, it should be taken to include such fragments.

The class marker is preferably labelled with a reporter, so that it is detectable.

### SPECIFICITY MARKER

In order to establish the binding ability of the polypeptide which is produced by a cell or colony, the polypeptide is exposed to one or more specificity markers. In general, the specificity marker comprises a binding partner of the polypeptide of interest, i.e., anything which has the ability to bind to the polypeptide of interest, and by doing so revealing a property of the polypeptide.

Preferably, the specificity marker comprises a specific binding partner of the polypeptide of interest. In such embodiments, the polypeptide of interest has a specific binding affinity for the specificity marker; in other words, the specificity marker is capable of being specifically bound by a polypeptide of interest.

Preferably substantially only the polypeptide of interest binds that specificity marker. Preferably, no other polypeptide, for example an unrelated polypeptide to the polypeptide of interest, binds that specificity marker. The specificity marker can therefore be anything that has the ability to bind to the polypeptide of interest, but does not substantially bind to other polypeptides.

The specificity marker may be comprised in, or derived from, a library of compounds. Such a library comprises a number of possible binders which may be selected for specific binding ability to the polypeptide of interest, by screening, for example.

Although at the minimum one specificity marker will be employed, it will be evident that the skilled person can use more than one specificity marker, i.e., two or more, or multiple specificity markers.

Multiple specificity markers may be employed for example in the case where a user wishes to detect two different antigens within the same class. Thus, for example, an animal may be immunised with two different antigens. Cells secreting antibodies to the different antigens may be detected and isolated by using two or more specificity markers conjugated to different fluorophores. As another example, multiple specificity markers may be used where it is desired to detect cells which are transfected with two or more different expression vectors, which may express transfected polypeptides or receptors. As a further example, the use of multiple specificity markers enables the detection of variants (i.e., mutants, etc) of a polypeptide of interest. In such a situation, the multiple specificity markers may have different specificity, one for each of the variants it is desired to detect.

Thus, where the singular term "specificity marker" is used in this document, it should be regarded as referring to embodiments in which a single specificity marker is employed, as well as those in which multiple, i.e., two or more, specificity markers are used (unless the context dictates otherwise).

Where more than specificity marker is employed, each specificity marker is capable of binding to and identifying one particular desired characteristic of the polypeptide of interest.

### COMBINED CLASS AND SPECIFICITY MARKER

As noted above, in some embodiments the class marker and the specificity marker may be provided in a single molecule.

Thus, in general, the methods described here may make use of a molecule or entity with a first part capable of functioning as a class marker, i.e., capable of identifying a class of polypeptides to which the polypeptide of interest belongs, and a second part capable of functioning as a specificity marker, i.e., capable of identifying a specific binding ability of the polypeptide of interest.

The molecule may comprise a class marker as described in this document attached to a specificity marker as described herein. The markers may be coupled, fused, mixed, combined, or otherwise joined to each other. The coupling, etc between the class marker and the specificity marker may be permanent or transient, and may involve covalent or non-covalent interactions (including ionic interactions, hydrophobic forces, Van der Waals interactions, etc). The exact mode of coupling is not important.

One example of a single molecule class and specificity marker is a fusion protein produced by expression of an in frame fusion of coding sequences for the class marker and the specificity marker. Another example is a chemical conjugate of a class marker, for example a polyclonal anti-IgG antibody, chemically coupled to a specificity marker, for example a peptide epitope of an antibody polypeptide of interest.

### LABEL / REPORTER / SIGNAL

At least one of the class marker and the specificity marker is capable of being detected, preferably by emitting a signal. For this purpose, the class marker or the specificity marker, or both, may be labelled with a reporter molecule.

In examples in which only one of the class marker and the specificity marker is labelled, it is preferably the specificity marker which is labelled.

A "signal", as used here, is any detectable event The signal may be the generation of an enzymatic activity, such as protease activity, transcriptional activity or luminescence inducing activity.

Preferably, however, the signal is emission or absorption of electromagnetic radiation, for example, light.

### FLUORESCENT LABEL

In highly preferred embodiments, the signal is a fluorescent signal. Included are fluorescence, phosphorescence or other signals which involve the modulation of the intensity or frequency of emission or absorption of radiation, for example, a FRET signal (described in further detail below).

Preferably, the fluorescent signal is emitted from a fluorophore such as a fluorescent protein or fluorescent chemical. Thus, the class marker or the specificity marker, or both, may comprise a reporter molecule comprising a fluorophore such as a fluorescent protein or fluorescent chemical.

Examples of fluorescent chemicals include allophycocyanine, phycocyanine, phycoerythrin, rhodamine, tetramethyl rhodamine, 7-nitro-benzofurazan rhodamine isothiocyanate, oxazine, coumarin, fluorescein derivatives, for example, FAM (6-carboxyfluorescein), TET (6-carboxy-4,7,2',7'-tetrachloro-fluorescein), (FITC) fluorescein isothiocyanate and carboxyfluorescein diacetate, as well as Texas Red, acridine yellow/orange, ethidium bromide, propidium iodide and bis-benzamide (commercially available from Hoechst under the trade name H33258).

Preferred fluorescent chemicals are fluorescein isothiocyanate, rhodamine and phycoerythrin, and preferred fluorescent proteins are Green Fluorescent Protein, Blue Fluorescent Protein, Cyan Fluorescent Protein, Yellow Fluorescent Protein and Red Fluorescent Protein. The fluorescent signal may be modulated by fluorescent resonance energy transfer (FRET).

Methods of conjugating fluorescent labels to various entities, including peptides, polypeptides and antibodies, are well known in the art.

The fluorescent signal may be emitted from a fluorescent polypeptide. Thus, the class marker or the specificity marker, or both, may comprise a reporter molecule comprising a fluorescent polypeptide.

Examples of fluorescent polypeptides and proteins include Green Fluorescent Protein (GFP) from *Aequorea victoria* and Red Fluorescent Protein (RFP) from *Discosoma* spp. Derivatives and variants of these proteins, such as Cyan Fluorescent Protein, Blue Fluorescent Protein, Enhanced Green Fluorescent Protein (EGFP; GFPmut1; Yang, T. T., et al. (1996) Nucleic Acids Res. 24(22):4592-4593;.Cormack, B. P., et al. (1996) Gene 173:33-38.), Enhanced Blue Fluorescent Protein (EBFP), Enhanced Yellow Fluorescent Protein (EYFP; Ormö, et al. (1996) Science 273:1392-1395), Destablised Enhanced Green Fluorescent Protein (d2EGFP; Living Colors Destabilized EGFP Vectors (April 1998) CLONTECHniques XIII(2):16-17), Enhanced Cyan Fluorescent Protein (ECFP), and GFPuv (Haas, J., et al. (1996) Curr. Biol. 6:315-324). may also be used. These fluorescent proteins are available from CLONTECH Laboratories, Inc. (Palo Alto, California, USA).

The signal may be a luminescence inducing activity. It will be appreciated that as light is generated during luminescence, the signal may at the same time be a luminescence inducing activity and emission of electromagnetic radiation.

The signal may also be the generation of an enzymatic activity, for example, transcriptional activity. The class marker or the specificity marker, or both, may therefore comprise a polypeptide with an assayable enzyme activity. Where the enzyme activity comprises transcriptional activity, this may be detected by assaying the expression of a reporter gene such as CD4, by fluorescent antibodies and FACs for example.

The reporter may be attached, coupled, fused, mixed, combined, or otherwise joined to the class or specificity marker. The attachment, etc between the reporter and the marker may be permanent or transient, and may involve covalent or non-covalent interactions (including hydrogen bonding, ionic interactions, hydrophobic forces, Van der Waals interactions, etc).

The reporter is permanently, preferably covalently attached to the class or specificity marker. In such preferred examples, the reporter is chemically coupled or cross-linked to the class or specificity marker. Any of the various methods of chemical coupling which are known in the art may be employed for this purpose.

It may be desirable to include spacing means between the reporter and the class or specificity marker. Such spacing means may suitably comprise linkers or spacers as known in the art. The purpose of the spacing means is to space the reporter and the marker, to avoid for example steric hindrance and to promote detection of the reporter and hence the marker. Accordingly, depending on the application, the use of shorter or longer spacers may be preferred.

The spacing means may comprise linkers or spacers which are polymers of differing lengths (the length of which may be controlled by controlling the degree of polymerisation). Numerous spacers and linkers are known in the art, and the skilled person will know how to choose and use these, depending on the application. The skilled person will also know what spacer length to use.

The spacers may be made for example of polyethylenglycol, PEG derivatives or polyalkanes or homo poly amino acids. Dextrans and dendrimers, as known in the art, may also be used. In particular, the linkers or spacers may comprise nucleotide polymers (nucleic acids, polynucleotides, etc) or amino acid polymers (proteins, peptides, polypeptides, etc).

### FRET Signal

The signal may comprise the emission or absorption of electromagnetic radiation, and the signal-generation molecules may be fluorophores. Particularly preferred are fluorescent molecules which participate in energy transfer (FRET).

FRET is detectable when two fluorescent labels which fluoresce at different frequencies are sufficiently close to each other that energy is able to be transferred from one label to the other. FRET is widely known in the art (for a review, see Matyus, 1992, J. Photochem. Photobiol. B: Biol., 12: 323-337). FRET is a radiationless process in which energy is transferred from an excited donor molecule to an acceptor molecule; the efficiency of this transfer is dependent upon the distance between the donor and acceptor molecules, as described below. Since the rate of energy transfer is inversely proportional to the sixth power of the distance between the donor and acceptor, the energy transfer efficiency is extremely sensitive to distance changes. Energy transfer is said to occur with detectable efficiency in the 1-10 nm distance range, but is typically 4-6 nm for favourable pairs of donor and acceptor.

Accordingly, the methods may be practised by choosing suitable pairs of donor and acceptor molecules, so that one of the class marker and the specificity marker comprises a donor molecule and the other of the class marker and the specificity marker comprises an acceptor molecule. When the markers bind to the polypeptide of interest, the donor molecule and the acceptor molecule are brought together so that energy transfer occurs.

Radiationless energy transfer is based on the biophysical properties of fluorophores. These principles are reviewed elsewhere (Lakowicz, 1983, Principles of Fluorescence Spectroscopy, Plenum Press, New York; Jovin and Jovin, 1989, Cell Structure and Function by Microspectrofluorometry, eds. E. Kohen and J.G. Hirschberg, Academic Press). Briefly, a fluorophore absorbs light energy at a characteristic wavelength. This wavelength is also known as the excitation wavelength. The energy absorbed by a fluorochrome is subsequently released through various pathways, one being emission of photons to produce fluorescence. The wavelength of light being emitted is known as the emission wavelength and is an inherent characteristic of a particular fluorophore. Radiationless energy transfer is the quantum-mechanical process by which the energy of the excited state of one fluorophore is transferred without actual photon emission to a second fluorophore. That energy may then be subsequently released at the emission wavelength of the second fluorophore. The first fluorophore is generally termed the donor (D) and has an excited state of higher energy than that of the second fluorophore, termed the acceptor (A). The essential features of the process are that the emission spectrum of the donor overlap with the excitation spectrum of the acceptor, and that the donor and acceptor be sufficiently close. The distance over which radiationless energy transfer is effective depends on many factors including the fluorescence quantum efficiency of the donor, the extinction coefficient of the acceptor, the degree of overlap of their respective spectra, the refractive index of the medium, and the relative orientation of the transition moments of the two fluorophores. In addition to having an optimum emission range overlapping the excitation wavelength of the other fluorophore, the distance between D and A must be sufficiently small to allow the radiationless transfer of energy between the fluorophores.

In a FRET assay, the fluorescent molecules are chosen such that the excitation spectrum of one of the molecules (the acceptor molecule) overlaps with the emission spectrum of the excited fluorescent molecule (the donor molecule). The donor molecule is excited by light of appropriate intensity within the donor's excitation spectrum. The donor then emits some of the absorbed energy as fluorescent light and dissipates some of the energy by FRET to the acceptor fluorescent molecule. The fluorescent energy it produces is quenched by the acceptor fluorescent molecule. FRET can be manifested as a reduction in the intensity of the fluorescent signal from the donor, reduction in the lifetime of its excited state, and re-emission of fluorescent light at the longer wavelengths (lower energies) characteristic of the acceptor. When the donor and acceptor molecules become spatially separated, FRET is diminished or eliminated.

Suitable fluorophores are known in the art, and include chemical fluorophores and fluorescent polypeptides, such as GFP and mutants thereof which fluoresce with different wavelengths or intensities (see WO 97/28261). Chemical fluorophores may be attached to immunoglobulin by incorporating binding sites therefor into the immunoglobulin during the synthesis thereof.

Preferably, however, the fluorophore is a fluorescent protein, which is advantageously GFP or a mutant thereof. GFP and its mutants may be synthesised together with the marker (where this is a polypeptide such as an immunoglobulin) by expression therewith as a fusion polypeptide, according to methods well known in the art. For example, a transcription unit may be constructed as an in-frame fusion of the desired GFP and the immunoglobulin, and inserted into a vector as described above, using conventional PCR cloning and ligation techniques.

### SOLID OR SEMI-SOLID MEDIA

In preferred embodiments, the polypeptide is secreted from a cell or colony of cells which is grown on the surface of or within solid or semi-solid media.

Growth of cells, particularly antibody secreting hybridomas, on such media enhances secretion, as described in Goding, J.W. 1980. Antibody production by hybridomas. [Review]. J. Immunol. Methods. 39(4): 285-308, Sharon, J., Morrison, S.L. and Kabat, E.A. 1979. Detection of specific hybridoma clones by replica immunoadsorption of their secreted antibodies. Proc. Natl. Acad. Sci. (USA). 76(3): 1420-4 and Davis, J.M., Pennington, J.E., Kubler, A.-M. and Conscience, J.F. 1982. A simple, single-step technique for selecting and cloning hybridomas for the production of monoclonal antibodies. J. Immunol. Methods. 50: 161-171.

Methylcellulose media may, for example, be obtained from Sigma-Aldrich Company Ltd (Dorset, UK) under catalogue number M0387 (Methyl cellulose viscosity 1,500 cP (2% aqueous solution, 20 °C) (lit.) CAS Number 9004-67-5) or catalogue number M0512 (Methyl cellulose viscosity 4,000 cP (2% aqueous solution, 20 °C) (lit.) CAS Number 9004-67-5).

In highly preferred embodiments, the polypeptide is secreted from a cell or colony of cells grown on the surface of or within methylcellulose media. The use of methylcellulose media is well known in the art, and protocols have been established to enable hybridoma cloning on such media. See for example, the ClonaCell™- HY Hybridoma Cloning Kit Procedure Manual (StemCell Technologies, Vancouver, Canada).

The media may optionally comprise growth factors or other supplements optimized to support the selection and growth of the relevant cells.

Where solid or semi-solid media are employed, it is preferred that either or both of the class marker and the specificity marker are included in the media to allow binding to take place. Where only one of the markers is included in the media, the other may be applied subsequently.

Application of the class marker limits the diffusion of the secreted polypeptide and causes the formation of a halo or aura of polypeptide surrounding the cell or cell colony. Formation of such a halo increases the effective concentration of the polypeptide in the vicinity of the cell or colony, to enable more efficient binding by the specificity marker to the polypeptide. Advantageously, therefore, our methods allow the application of a smaller amount or concentration of specificity marker compared to methods which do not make use of a class marker.

### HALO / AURA

In preferred embodiments, the polypeptide forms a halo or aura around the cell or colony which produces it.

In general, with such a halo or aura, the polypeptide is present in an area or zone which is associated with, coincident with, or around the cell or colony. Preferably, the aura or halo extends beyond the boundaries of the cell or colony. Preferably, the aura or halo as defined by the presence of the polypeptide extends to 1, 2, 3, 4, 5, 10, 20 or 30 or more colony diameters beyond the boundaries of the colony.

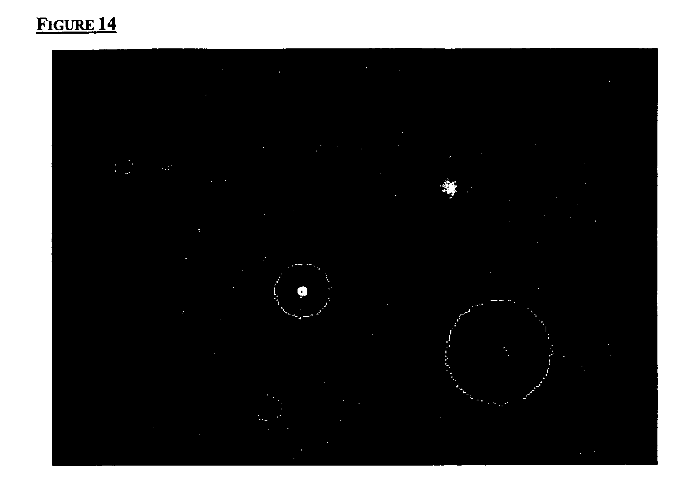

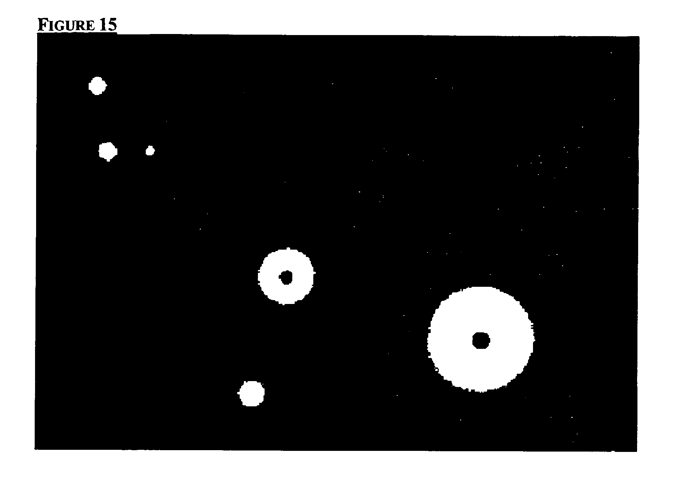

The aura or halo is detected by imaging techniques known in the art, and described in for example, US Patent Application Numbers 11/050,826 and 11/050,818 filed January 27, 2005 (D Young & Co Attorney references P021480US and P021481US). Examples of visualised haloes are shown in Figure 14, while Figure 15 shows the image of Figure 14 after processing by the image processing

The halo or aura is detected as the presence of signal strength deriving from polypeptide existing beyond and between signals of the colony and the background. Alternatively, or in addition, the halo or aura around a colony may be detected as the presence of a signal strength significantly above background which is coincident with, but also extending beyond, the signal associated with the colony.

In preferred embodiments, polypeptide is secreted into the medium such that it surrounds the cell or colony to form a halo or aura. Thus, the halo or aura in general terms comprises a concentration of polypeptide in the immediate environs of the cell or colony.

Haloes or auras are particularly pronounced so where the cell or colony is growing on the surface of or within a solid or semi-solid medium. Preferably, the halo or aura arises through the fact that diffusion of the secreted polypeptide away from the cell or colony producing it is restricted. More preferably, reaction with the class marker promotes restriction of diffusion to enable the formation of a halo or aura of secreted polypeptide around the cell or colony. The class marker may specifically bind to the polypeptide to aggregate it or promote formation of a suspension, and / or the precipitation out of solution of the polypeptide.

The formation of a of polypeptide / class marker complex in the form of the aura or halo increases the effective concentration of the polypeptide in the particular area. Preferably, such an increased effective concentration enables more efficient binding by the specificity marker to the polypeptide. Advantageously, therefore, the amount of specificity marker which is required to be applied in the methods as described here is less than in the absence of application of the class marker.

The halo or aura may be detected visually without any aids, or detection may be promoted by reaction with the class marker. For example, where the class marker is labelled with a signal generating reporter, the halo or aura may take the form of an area of signal surrounding the cell or colony. In particular embodiments, the label is a fluorescence label and the aura or halo comprises an area of fluorescence. Formation of such a halo or aura allows the detection of secreted polypeptide to be more easily detected.

The halo or aura may preferably be detected by a visualisation system in a robotic picking apparatus, for example.

### IGG / ANTIBODY POLYPEPTIDE OF INTEREST

In a highly preferred embodiment, the polypeptide of interest comprises an antibody or immunoglobulin. Specifically, we provide a method of detecting a polypeptide of interest comprising an antibody or immunoglobulin produced by a cell or cell colony.

The cell or cell colony may therefore comprise an antibody producing cell, preferably an antibody secreting cell, such as a B-cell, transfected myeloma or a hybridoma. The class of polypeptides which is detectable by the class marker may therefore preferably comprise an antibody class or an antibody isotype of interest. In preferred embodiments, the antibody class comprises an Ig class such as an IgG class, an IgA class, an IgM class, an IgD class or an IgE class.

The polypeptide class may comprise an immunoglobulin subclass. For example, the polypeptide class may comprise an IgG subclass including an IgG1 subclass, an IgG2 subclass, an IgG3 subclass or an IgG4 subclass. The polypeptide class may comprise an IgA subclass such as an IgA1 subclass or an IgA2 subclass.

In a preferred embodiment, the class marker is capable of identifying an antibody class, and may comprise an Ig specific binding agent, preferably an Ig specific antibody. Such antibodies may be directed against any suitable common motif or sequence present in the Ig class of interest. Examples of such common motifs and sequences include the constant regions of a heavy chain, including gamma (γ), alpha (α), mu (µ), delta (δ) and epsilon (ε) regions. Other regions suitable for use include constant regions of a light chain, including lambda (λ) and kappa (κ) regions.

In preferred embodiments, the class marker comprises a binding agent capable of binding to an IgG class such as an anti-IgG antibody, preferably a specific anti-IgG antibody. Thus, for example, a class marker specific for the IgG isotype may be generated in the form of an antibody (from another species) raised against a γ chain.

Antibodies specific for antibody Ig classes are well known in the art and are available commercially, as set out in the section below.

In preferred embodiments, the class marker comprises a polyclonal antibody. In such preferred embodiments, the class marker comprises polyclonal anti-mouse or polyclonal anti-human IgG antibody. Preferably, the class marker comprises a goat polyclonal anti-mouse IgG or a goat polyclonal anti-human IgG antibody. The antibody may comprise a whole antibody, or it may comprise a fragment thereof, preferably with binding ability, such as a F(ab')₂ fragment.

In order to establish the binding ability of the antibody which is produced, the antibody is exposed to a specificity marker. In general, the specificity marker comprises a binding partner of the antibody of interest, i.e., anything which has the ability to bind to the antibody of interest.

Preferably, the specificity marker comprises a specific binding partner of the antibody of interest. In such embodiments, the antibody of interest is specific for the specificity marker; in other words, the specificity marker is capable of being specifically bound by an antibody of interest.

Preferably substantially only the antibody of interest binds that specificity marker. Preferably, no other antibody binds that specificity marker. The specificity marker can therefore be anything that has the ability to bind to the antibody of interest, but does not substantially bind to other antibodies.

The specificity marker comprises at least an epitope of the antibody of interest. It may comprise an antigen of the antibody of interest. For example a polypeptide or other entity used to generate the antibody of interest may be employed. Such a polypeptide may comprise a polypeptide which is exposed to B-cells, or injected into an animal such as a rabbit, goat, rat or mouse for the purpose of generating antibodies.

The specificity marker may comprise any portion of the antigen capable of binding to the specificity marker. The specificity marker may therefore comprise a fragment of the antigen, preferably an immunogenic fragment of the antigen. Fragments and portions may be generated by means known to the person skilled in the art, for example by protease digestion of an antigen. Furthermore, synthetic proteins and peptides having the required binding ability may be easily synthesised.

### CLASS MARKERS SPECIFIC FOR IG CLASSES

### IgG Specific Class Markers

Anti-human Ig antibodies include Human IgG Pan antibody Fc specific (Cat# 3010-1), Human IgG1k Fab'2, specific (Cat# 3011-1), Human IgG1 (1+k), Fc, specific (Cat# 3012-1), Human IgGk light chain, Fab'2, specific (Cat# 3013-1), Human IgG 2k3k A M Fab'2 (Cat# 3014-1), available from Epitomics, Inc (Burlingame, California, USA).

Anti-IgG antibodies may also be obtained from DakoCytomation (Glostrup, Denmark), as shown in the table below.

| **Cat No.** | **Description** | **Source** | **Notes / Label** |
|---|---|---|---|
| A0424 | IgG, Specific for Gamma-Chains | Polyclonal, Rabbit | Ig fraction |
| Q0331 | IgG, Specific for Gamma-Chains | Polyclonal, Rabbit | Ig fraction |
| O9116 | IgG, Specific for Gamma-Chains | Polyclonal, Rabbit | Affinity isolated |
| A0407 | IgG, Specific for Gamma-Chains | Polyclonal, Rabbit | F(ab')2 fragment |
| Q8331 | IgG, Specific for Gamma-Chains | Polyclonal, Rabbit | Ready-to-use |
| A0473 | IgG, Specific for Gamma-Chains | Polyclonal, Gt | lg fraction |
| O4418 | IgG, for removal of IgG (IgM detection) | Polyclonal, Gt | Ig fraction |
| D0336 | IgG, Specific for Gamma-Chains | Polyclonal, Rabbit | AP conjugated |
| F0202 | IgG, Specific for Gamma-Chains | Polyclonal, Rabbit | FITC conjugated |
| P0214 | IgG, Specific for Gamma-Chains | Polyclonal, Rabbit | HRP conjugated |
| P0406 | IgG, Specific for Gamma-Chains | Polyclonal, Rabbit | HRP conjugated, F(ab')2 fragment |

### IgA Specific Class Markers

Anti-IgA antibodies may be obtained from DakoCytomation (Glostrup, Denmark), as shown in the table below.

| **Cat No.** | **Description** | **Source** | **Notes / Label** |
|---|---|---|---|
| A0092 | IgA, Specific for Alpha-Chains | Polyclonal, Rabbit | Ig fraction |
| Q0332 | IgA, Specific for Alpha-Chains | Polyclonal, Rabbit | Ig fraction |
| Q8332 | IgA, Specific for Alpha-Chains | Polyclonal, Rabbit | Ready-to-use |
| A0474 | IgA, Specific for Alpha-Chains | Polyclonal, Goat | Ig fraction |
| A0408 | IgA, Specific for Alpha-Chains | Polyclonal, Rabbit | Ig fraction F(ab')2 fragment |
| D0338 | IgA, Specific for Alpha-Chains | Polyclonal, Rabbit | AP conjugated |
| F0204 | IgA, Specific for Alpha-Chains | Polyclonal, Rabbit | FITC conjugated |
| P0216 | IgA, Specific for Alpha-Chains | Polyclonal, Rabbit | HRP conjugated |
| P0405 | IgA, Specific for Alpha-Chains | Polyclonal, Rabbit | HRP conjugated F(ab')2 fragment |

### IgD Specific Class Markers

Anti-IgD antibodies may be obtained from DakoCytomation (Glostrup, Denmark), as shown in the table below.

| **Cat No.** | **Description** | **Source** | **Notes / Label** |
|---|---|---|---|
| A0093 | IgD, Specific for Delta-Chains | Polyclonal, Rabbit | Ig fraction |
| M0703 | IgD, Specific for Delta-Chains, IgD26 | Monoclonal, mouse | Supernatant |

### IgE Specific Class Markers

Anti-IgE antibodies may be obtained from DakoCytomation (Glostrup, Denmark), as shown in the table below.

| | | | |
|---|---|---|---|
| **Cat No.** | **Description** | **Source** | **Notes / Label** |
| A0094 | IgE, Specific for Epsilon-Chains | Polyclonal, Rabbit | Ig fraction |
| M0793 | IgE, E-1 | Monoclonal, Mouse | Supernatant |

### IgM Specific Class Markers

Anti-IgM antibodies may be obtained from DakoCytomation (Glostrup, Denmark), as shown in the table below.

| **Cat No.** | **Description** | **Source** | **Notes / Label** |
|---|---|---|---|
| A0426 | IgM, Specific for Mu-Chains | Polyclonal, Rabbit | lg fraction |
| Q0333 | IgM, Specific for Mu-Chains | Polyclonal, Rabbit | Ig fraction |
| Q8333 | IgM, Specific for Mu-Chains | Polyclonal, Rabbit | Ready-to-use |
| A0475 | IgM, Specific for Mu-Chains | Polyclonal, Goat | Ig fraction |
| D0337 | IgM, Specific for Mu-Chains | Polyclonal, Rabbit | AP conjugated |
| F0203 | IgM, Specific for Mu-Chains | Polyclonal, Rabbit | FITC conjuugated |
| P0215 | IgM, Specific for Mu-Chains | Polyclonal, Rabbit | HRP conjugated |
| P0322 | IgM, Specific for Mu-Chains | Polyclonal, Rabbit | HRP conjugated, F(ab')2 fragment |

### RECEPTOR POLYPEPTIDE OF INTEREST

According to another preferred embodiment, the polypeptide of interest comprises an receptor polypeptide. Specifically, we provide a method of detecting a polypeptide of interest comprising a receptor produced by a cell or cell colony.

A receptor, as the term is used in this document, means any polypeptide which is capable of binding another molecule, preferably a small molecule such as a ligand. Preferably, a receptor is a protein molecule that receives and responds to a specific neurotransmitter, hormone, ligand or other substance. Preferably, the receptor is capable of binding an affinity ligand of the receptor.

Where the polypeptide comprises a receptor, the cell or cell colony may be a cultured cell which has been engineered to express the receptor, preferably as a recombinant protein. The cell which is transfected may be any suitable cell as known in the art, for example, suspension adapted adherent cells such as CHO-S are suitable.

The cell may be transfected with an expression vector encoding a receptor polypeptide. The receptor preferably comprises a trans-membrane receptor, and may be a peripheral membrane receptor, a transmembrane protein receptor or an intracellular receptor, such as a nuclear receptor. The class of polypeptides which is detectable by the class marker may therefore preferably comprise a receptor class or subclass, such as a 7TM receptor class.

The receptor may comprise a G protein-coupled receptor (GPCR), also known as a seven transmembrane receptor or 7TM receptor. For example, the receptor may comprise any of the following (ligands in brackets following): a "muscarinic" acetylcholine receptor (acetylcholine and muscarine), an adenosine receptor (adenosine), an adrenoceptor or adrenergic receptor (ligand: adrenaline, and other structurally related hormones and drugs), a GABA receptor, type-b (γ-aminobutyric acid or GABA), an angiotensin receptor (angiotensin), a cannabinoid receptor (cannabinoids), a cholecystokinin receptor (cholecystokinin), a dopamine receptor (dopamine), a glucagon receptor (glucagon), a histamine receptor (histamine), a olfactory receptor, a opioid receptor (opioids), a rhodopsin (a photoreceptor), a secretin receptor (secretin), a serotonin receptors (Serotonin, also known as 5-Hydroxytryptamine or 5-HT) or a somatostatin receptor (Somatostatin).

The receptor may comprise a tyrosine kinase receptor, such as an erythropoietin receptor (Erythropoietin), an insulin receptor (Insulin), a growth factor receptor or a cytokine receptor. The receptor may comprise a guanylyl cyclase receptor such as GC-A & GC-B, comprising receptors for Atrial-natriuretic peptide (ANP) and other natriuretic peptides or GC-C, a guanylin receptor

The receptor may comprise an ionotropic receptor, for example a nicotinic acetylcholine receptor (Acetylcholine, Nicotine), a glycine receptor (GlyR) (Glycine, Strychnine), a GABA receptor: GABA-A, GABA-C (GABA), a glutamate receptor, an NMDA receptor, an AMPA receptor, a kainate receptor (Glutamate) or a 5-HT3 receptor (Serotonin).

In some embodiments, either or both of the class marker and the specificity marker comprises a ligand of the receptor. Preferably, however, the class marker comprises a binding agent capable of binding to members of a receptor class, such as an antibody capable of binding to members of a 7TM receptor class. The specificity marker preferably comprises a ligand of the receptor.

In preferred embodiments, the ligand comprises an affinity ligand of the receptor. The ligand may comprise a small molecule, by which term we mean a molecule which has a molecular weight below 50 kDa, more preferably below 25 kDa, more preferably below 10 kDa, more preferably below 1 kDa, more preferably below 500 Da, most preferably below 25 Da. Preferably, the ligand comprises an organic molecule. The ligand may comprise any of the known biomolecules which exist in organisms, such as hormones, peptides, amino acids, nucleic acids, etc. The ligand may be synthetic or natural. In highly preferred embodiments, the ligand comprises a synthetic small molecule.

### ANTIBODIES

In preferred embodiments, the class marker and/or specificity marker comprises an antibody.

For example, the class marker may comprise an antibody capable of binding to and identifying a class of polypeptides. Such an antibody may be capable of binding to a consensus sequence or motif shared by polypeptides in that class. Alternatively, or in addition, the specificity marker may comprise an antibody capable of specifically binding to the polypeptide of interest and thereby identifying it by virtue of the binding.

Antibodies comprise immunoglobulin molecules. Immunoglobulin molecules are in the broadest sense members of the immunoglobulin superfamily, a family of polypeptides comprising the immunoglobulin fold characteristic of antibody molecules, which contains two β sheets and, usually, a conserved disulphide bond. Members of the immunoglobulin superfamily are involved in many aspects of cellular and non-cellular interactions *in vivo,* including widespread roles in the immune system (for example, antibodies, T-cell receptor molecules and the like), involvement in cell adhesion (for example the ICAM molecules) and intracellular signalling (for example, receptor molecules, such as the PDGF receptor). The methods described here may therefore make use of any immunoglobulin superfamily molecule which is capable of binding to a target polypeptide of interest. Peptides or fragments derived from immunoglobulins may also be used.

Antibodies, as used herein, refers to complete antibodies or antibody fragments capable of binding to a selected target, and including Fv, ScFv, F(ab') and F(ab')₂, monoclonal and polyclonal antibodies, engineered antibodies including chimeric, CDR-grafted and humanised antibodies, and artificially selected antibodies produced using phage display or alternative techniques. Small fragments, such as Fv and ScFv, possess advantageous properties for diagnostic and therapeutic applications on account of their small size and consequent superior tissue distribution. Preferably, the antibody is a single chain antibody or ScFv.

The antibodies may be altered antibodies comprising an effector protein such as a toxin or a label. Use of labelled antibodies allows the imaging of the distribution of the antibody *in vivo.* Such labels may be radioactive labels or radioopaque labels, such as metal particles, which are readily visualisable within the body of a patient. Moreover, they may be fluorescent labels (such as the ones described here) or other labels which are visualisable on tissue samples removed from patients. Antibodies with effector groups may be linked to any association means as described above.

Antibodies may be obtained from animal serum, or, in the case of monoclonal antibodies or fragments thereof, produced in cell culture. Recombinant DNA technology may be used to produce the antibodies according to established procedure, in bacterial, yeast, insect or preferably mammalian cell culture. The selected cell culture system preferably secretes the antibody product.

Growing of hybridoma cells or mammalian host cells in vitro is carried out in suitable culture media, which are the customary standard culture media, for example Dulbecco's Modified Eagle Medium (DMEM) or RPMI 1640 medium, optionally replenished by a mammalian serum, for example foetal calf serum, or trace elements and growth sustaining supplements, for example feeder cells such as normal mouse peritoneal exudate cells, spleen cells, bone marrow macrophages, 2-aminoethanol, insulin, transferrin, low density lipoprotein, oleic acid, or the like. The culture medium may be serum-free or animal-produce free, such as a chemically defined medium, in order to minimise animal derived contamination. Multiplication of host cells which are bacterial cells or yeast cells is likewise carried out in suitable culture media known in the art, for example for bacteria in medium LB, NZCYM, NZYM, NZM, Terrific Broth, SOB, SOC, 2 x YT, or M9 Minimal Medium, and for yeast in medium YPD, YEPD, Minimal Medium, or Complete Minimal Dropout Medium.

Use of insect cells as hosts for the expression of proteins has advantages in that the cloning and expression process is relatively easy and quick. In addition, there is a high probability of obtaining a correctly folded and biologically active protein when compared to bacterial or yeast expression. Insect cells may be cultured in serum free medium, which is cheaper and safer compared to serum containing medium. Recombinant baculovirus may be used as an expression vector, and the construct used to transfect a host cell line, which may be any of a number of lepidopteran cell lines, in particular *Spodoptera frugiperda Sf9*, as known in the art. Reviews of expression of recombinant proteins in insect host cells are provided by Altmann et al. (1999), Glycoconj J 1999, 16, 109-23 and Kost and Condreay (1999), Curr Opin Biotechnol, 10, 428-33.

*In vitro* production provides relatively pure antibody preparations and allows scale-up to give large amounts of the desired antibodies. Techniques for bacterial cell, yeast, insect and mammalian cell cultivation are known in the art and include homogeneous suspension culture, for example in an airlift reactor or in a continuous stirrer reactor, or immobilised or entrapped cell culture, for example in hollow fibres, microcapsules, on agarose microbeads or ceramic cartridges.

Large quantities of the desired antibodies can also be obtained by multiplying mammalian cells *in vivo.* For this purpose, hybridoma cells producing the desired antibodies are injected into histocompatible mammals to cause growth of antibody-producing tumours. Optionally, the animals are primed with a hydrocarbon, especially mineral oils such as pristane (tetramethyl-pentadecane), prior to the injection. After one to three weeks, the antibodies are isolated from the body fluids of those mammals. For example, hybridoma cells obtained by fusion of suitable myeloma cells with antibody-producing spleen cells from Balb/c mice, or transfected cells derived from hybridoma cell line Sp2/0 that produce the desired antibodies are injected intraperitoneally into Balb/c mice optionally pre-treated with pristane, and, after one to two weeks, ascitic fluid is taken from the animals.

The foregoing, and other, techniques are discussed in, for example, Kohler and Milstein, (1975) Nature 256:495-497; US 4,376,110; Harlow and Lane, Antibodies: a Laboratory Manual, (1988) Cold Spring Harbor. Techniques for the preparation of recombinant antibody molecules is described in the above references and also in, for example, EP 0623679; EP 0368684 and EP 0436597.

The cell culture supernatants are screened for the desired antibodies, preferentially by immunofluorescent staining of cells expressing the desired target by immunoblotting, by an enzyme immunoassay, for example a sandwich assay or a dot-assay, or a radioimmunoassay.

For isolation of the antibodies, the immunoglobulins in the culture supernatants or in the ascitic fluid may be concentrated, for example by precipitation with ammonium sulphate, dialysis against hygroscopic material such as polyethylene glycol, filtration through selective membranes, or the like. If necessary and/or desired, the antibodies are purified by the customary chromatography methods, for example gel filtration, ionexchange chromatography, chromatography over DEAE-cellulose and/or immunoaffinity chromatography, for example affinity chromatography with the a protein containing a target or with Protein-A.

Antibodies generated according to the foregoing procedures may be cloned by isolation of nucleic acid from cells, according to standard procedures. Usefully, nucleic acids variable domains of the antibodies may be isolated and used to construct antibody fragments, such as scFv.

The methods described here preferably employ recombinant nucleic acids comprising an insert coding for a heavy chain variable domain and/or for a light chain variable domain of antibodies. By definition such nucleic acids comprise coding single stranded nucleic acids, double stranded nucleic acids consisting of the coding nucleic acids and of complementary nucleic acids thereto, or these complementary (single stranded) nucleic acids themselves.

Furthermore, nucleic acids encoding a heavy chain variable domain and/or for a light chain variable domain of antibodies can be enzymatically or chemically synthesised nucleic acids having the authentic sequence coding for a naturally-occurring heavy chain variable domain and/or for the light chain variable domain, or a mutant thereof. A mutant of the authentic sequence is a nucleic acid encoding a heavy chain variable domain and/or a light chain variable domain of the above-mentioned antibodies in which one or more amino acids are deleted or exchanged with one or more other amino acids. Preferably the modification(s) are outside the complementary determining regions (CDRs) of the heavy chain variable domain and/or of the light chain variable domain of the antibody. Such a mutant nucleic acid is also intended to be a silent mutant wherein one or more nucleotides are replaced by other nucleotides with the new codons coding for the same amino acid(s). Such a mutant sequence is also a degenerated sequence. Degenerated sequences are degenerated within the meaning of the genetic code in that an unlimited number of nucleotides are replaced by other nucleotides without resulting in a change of the amino acid sequence originally encoded. Such degenerated sequences may be useful due to their different restriction sites and/or frequency of particular codons which are preferred by the specific host, particularly yeast, bacterial or mammalian cells, to obtain an optimal expression of the heavy chain variable domain and/or a light chain variable domain.

The term mutant is intended to include a DNA mutant obtained by *in vitro* or *in vivo* mutagenesis of DNA according to methods known in the art.

Recombinant DNA technology may be used to improve antibodies. Thus, chimeric antibodies may be constructed in order to decrease the immunogenicity thereof in diagnostic or therapeutic applications. Moreover, immunogenicity may be minimised by humanising the antibodies by CDR grafting [European Patent 0 239 400 (Winter)] and, optionally, framework modification [European Patent 0239400; Riechmann et al., (1988) Nature 322:323-327; and as reviewed in international patent application WO 90/07861 (Protein Design Labs)].

Recombinant nucleic acids may be employed comprising an insert coding for a heavy chain variable domain of an antibody fused to a human constant domain γ, for example γ1, γ2, γ3 or γ4, preferably γ1 or γ4. Likewise recombinant DNAs comprising an insert coding for a light chain variable domain of an antibody fused to a human constant domain κ or λ, preferably κ may also be used.

More preferably, CDR-grafted antibodies, which are preferably CDR-grafted light chain and heavy chain variable domains only, may be used. Advantageously, the heavy chain variable domain and the light chain variable domain are linked by way of a spacer group, optionally comprising a signal sequence facilitating the processing of the antibody in the host cell and/or a DNA coding for a peptide facilitating the purification of the antibody and/or a cleavage site and/or a peptide spacer and/or an effector molecule. Such antibodies are known as ScFvs.

Antibodies may moreover be generated by mutagenesis of antibody genes to produce artificial repertoires of antibodies. This technique allows the preparation of antibody libraries, as discussed further below; antibody libraries are also available commercially. Hence, artificial repertoires of immunoglobulins, preferably artificial ScFv repertoires, are used as an immunoglobulin source.

Isolated or cloned antibodies may be linked to other molecules, for example nucleic acid or protein association means by chemical coupling, using protocols known in the art (for example, Harlow and Lane, Antibodies: a Laboratory Manual, (1988) Cold Spring Harbor, and Maniatis, T., Fritsch, E. F. and Sambrook, J. (1991), Molecular Cloning: A Laboratory Manual. Cold Spring Harbor, New York, Cold Spring Harbor Laboratory Press).

### ROBOTIC DETECTION AND PICKING

Any of the steps set out in relation to the detection method, such as exposing the polypeptide to a marker, detection of binding, as well as associated steps such as selection and/or picking of cells or colonies of interest may be conducted using automated robotic apparatus. In preferred examples, the robotic apparatus comprises a ClonePix FL apparatus (Genetix, New Milton, United Kingdom).

Features of a robotic apparatus which are advantageous for the performance of the methods described here, and which are present in the ClonePixFL apparatus, include any one or more of the following: cool white light illumination; up to 5 fluorescence combinations; high-resolution cooled CCD camera; ability to image at standard pixel resolution of 7µm permitting fluorescent detection of colonies with as few as 10 cells; image zooming to 1 µm resolution for detailed colony inspection; ability to pick colonies at up to 400 clones per hour; easy-to-use custom software (ExCellerate) for intelligent picking, Halo Recognition, barcoding and clone-by-clone data tracking; stackers hold up to 10 source and collection plates, and optional Class II-type containment.

The ClonePixFL apparatus is now described in more detail.

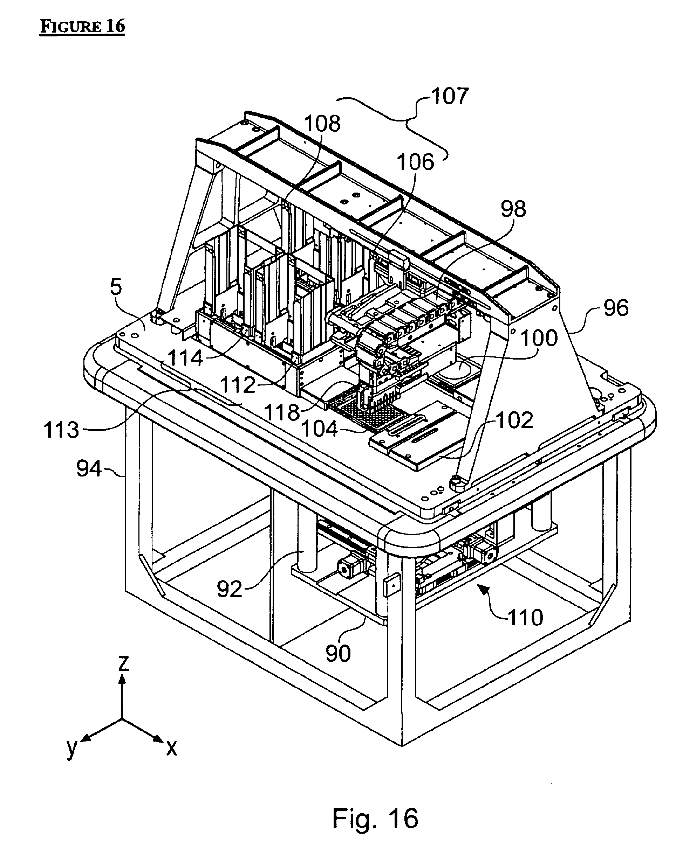

Figure 16 is a perspective view of the ClonePixFL robotic platform for carrying out methods of the invention in an automated way. The apparatus may be considered to be a robot for picking, gel coring or other biological manipulation task with integrated fluorescence excitation and collection (i.e. detection) optics. The apparatus can be subdivided notionally into two half spaces existing above and below a main bed 5 which is supported by a frame 94.

Above the main bed 5, the apparatus appears as similar to a conventional picking robot. A cell picking head 118 is provided that comprises a plurality of hollow pins for aspirating animal cells. The cell picking head 118 is movable over the main bed 5 by a head position system made up of x- y- and z-linear positioners 98 connected in series and suspended from a gantry 96. A wash/dry station 102 is also provided on the main bed 5 for cleansing the pins. The whole upper half space of the apparatus will typically be enclosed in a housing (not shown) including a hinged door extending over one side and part of the top of the apparatus.

Below the main bed 5, an optics sub-assembly 110 is provided to accommodate fluorescence excitation and detection optics system which is mounted on a tray 90 suspended from the main bed 5 by pillars 92. The under-slung optics system is arranged to view containers such as Petri dishes and well plates placed on the imaging station 100.

In use in the performance of the detection method described in this document, a Petri dish or other container (not shown) containing cells or colonies producing polypeptides (some of interest) is placed on the imaging station 100. Such containers are referred to for convenience generically as "well plate"s in the following description, although it will be evident that they need not comprise wells. Thus, where the term "plate" or "well plate" is employed, it should be understood as encompassing any container suitable for growing cells, such as Petri dishes, microtitre dishes, 6 well plates, etc.

The main bed 5 is provided with two main working stations, namely an imaging station 100 and a replating station 104, each of which is positioned at the end of a respective well plate feed lane. Each well plate feed lane has a well plate feeder/stacker. The well plate feeder/stacker 107 for the imaging station 100 has a well plate feed storage cassette 106 and well plate (re-)stack storage cassette 108. A stack of well plates are held

in the feed storage cassette 106, fed in turn down the lane via a delidder (not shown) to the imaging station 100, returned back along the lane, relidded and passed into the rear storage cassette 108. A similar well plate feeder/stacker 113 is used for the other lane to supply well plates from the storage cassette 112 to the replating station 104 and back along the lane to the (re-)stack storage cassette 114.

The well plate feeder/stacker mechanisms including delidding are described fully in EP-A-1 293 783.

It should be noted that although the description above refers the term "well plate" in the description of the "well plate feed lane at the end of the imaging station 100", the "well plate feeder/stacker 107" and the "well plate feed storage cassette 106", the term "well plate" should be taken as limiting to a container with wells. Instead, it should be treated as a generic description of any container capable of containing cells to be picked. In the performance of the detection method described herein, for example, it will be preferable to use Petri dishes or other flat dishes for growing cells producing polypeptides to be picked. Accordingly, such dishes may be used in the apparatus described with appropriate minor modifications, if necessary.

The cell picking head 118 can thus be moved from the imaging station to the replating station to allow replating of animal cells from a target well plate to a destination well plate. The arrangement described above enables a plurality of target plates, each containing cells or colonies expressing polypeptides to be picked, in containers such as Petri dishes, to be processed at the imaging station 100 in turn. The picked cells or colonies are plated onto destination well plates at the replating station 104.

In the illustrated example, there is only one destination lane. However, it may be desirable in some cases to have 2, 3 or 4 destination lanes. This may be useful when it is desired to split the animal cells from a given target well into multiple destination wells. The feeder/stacker mechanism is fully modular, so the number of well plate feed lanes can be increased without difficulty.

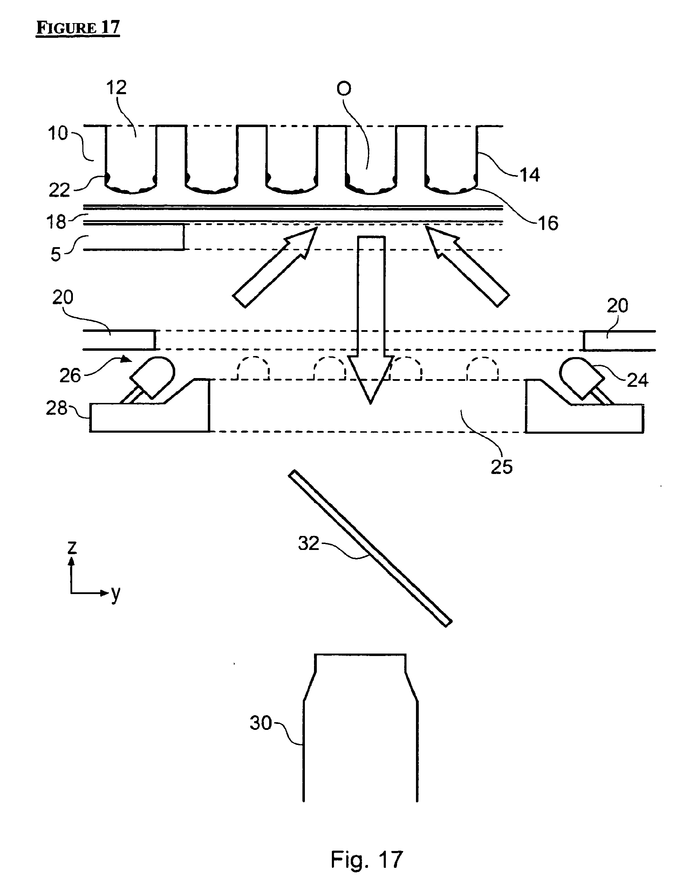

Figure 17 is a schematic sectional side view showing principles of the design of the optical sub-assembly 110. Part of a well plate 10 showing 5 wells is also shown. Adherent colonies 22 have been cultured in the wells also as shown, the colonies forming around the base 16 and lower sidewalls 14 of the wells 12. It will be appreciated that samples in other containers may also be studied, such as Petri dishes described above. In such containers, particularly those which contain semi-solid media such as methylcellulose, cells and colonies are growing in the media.

The imaging station is formed in an aperture in the main bed 5 covered by a sheet of optically transparent material, typically glass, that forms a light table 18. For optical analysis, a well plate 10 is arranged on the light table 18 as shown, having been deposited there by the well plate feeder/stacker. The apparatus is designed to image one well at a time. To image a specific well 12 of a well plate, the optical sub-assembly 110 is aligned relative to the well 12.

The optical sub-assembly 110 comprises two illumination sources and a collection part.

The first illumination source is formed of a plurality of white light emitting diodes (LEDs) 24 arranged to form an LED ring 26 located in a collar 28 with a central aperture 25 with the optical axes of the LEDs lying on the surface of a common cone, the point of which is coincident and labeled as the object position O in the figure. This white light source is provided principally to collect conventional images of the sample, for example as are used for performing cell confluence detection by image processing techniques. An apertured top plate 20 lying above the LED ring 26 is also illustrated. This is a structural component and has no significance for the optical design.

This second illumination source (not shown in this figure) is arranged to illuminate from the side, as shown by the sideways arrow, onto a semi-silvered mirror 32 which deflects the excitation light vertically onto the sample, as shown by the upwardly pointing arrow, in order to perform fluorescence measurements.

The collection part of the optical sub-assembly is made up of a zoom lens 30 with autofocus and is used to collect light when either (or both) of the illumination sources is used. The optical axis is vertical and coincident with the object position O.

The well to be imaged is thus aligned laterally with the optical axis of the collection optics and the fluorescence excitation optics and laterally and vertically with the center point of the white light lateral illumination, whereby the center point of the lateral illumination is around the base of the well or slightly higher as illustrated. The LEDs 24 thus illuminate a well 12 arranged in the object position O at an oblique angle from below so that an image of the well 12 is taken in a dark field configuration where light from the LEDs, if not scattered, does not contribute to the well image gathered by the collection lens 30.

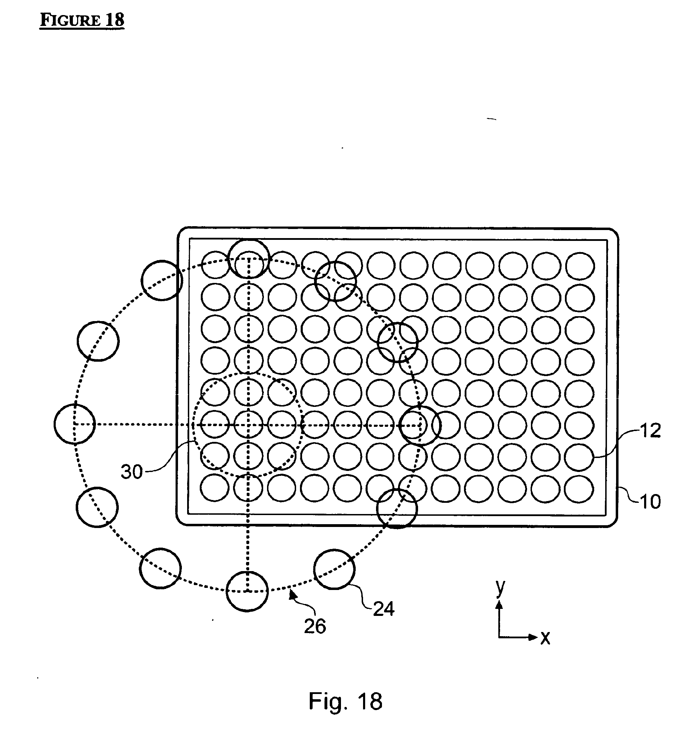

Figure 18 is a schematic plan view of selected parts of the optical system shown in Figure 17. The well plate 10 is a 96 well version and is shown aligned with the optical sub-assembly 110 so that a well 12 three rows up (row m=3) and two columns along (column n=2) is targeted, as illustrated by the objective lens 30 and LED ring 26 of LEDs 24. The optical sub-assembly is arranged on x- and y-positioners so that the collection lens 30 and illumination ring 26 can be moved together to image any one of the wells 12. Typically, the wells will be imaged in sequence row-wise and column-wise with a rastering process. This is achieved by moving the optical sub-assembly while the well plate remains static which is preferable so that liquid in the wells is not shaken by moving the well plate between imaging each well which might have an adverse influence on the imaging.

It will be appreciated that the ability of the apparatus to image a growing container comprising a single well may be extended to enable imaging of a growing container of any suitable size. Thus, samples in other (larger) containers may also be studied, such as Petri dishes described above suitable for use in the detection method described in this document. In such dishes, the cells or colonies will be scattered more or less randomly across the surface of the plate, instead of being arranged in a row / column configuration. Nevertheless, the x- and y-positioners do not restrain the collection lens 30 and illumination ring 26 to movement in a discrete fashion, but these are instead movable continuously across the surface of the plate. Accordingly, the x- and y- positioners enable any portion of the plate to be imaged by the collection lens 30 and illumination ring 26.

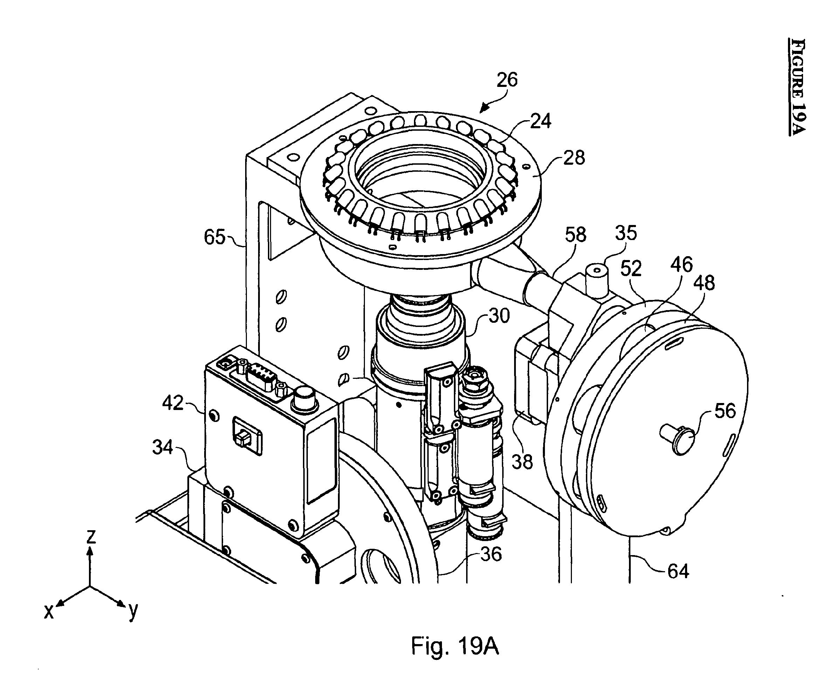

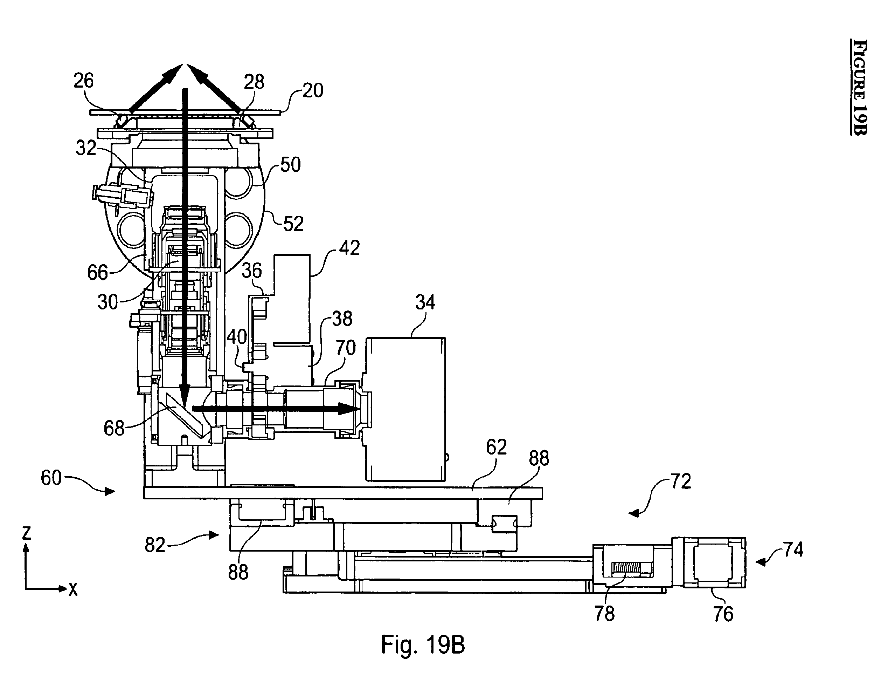

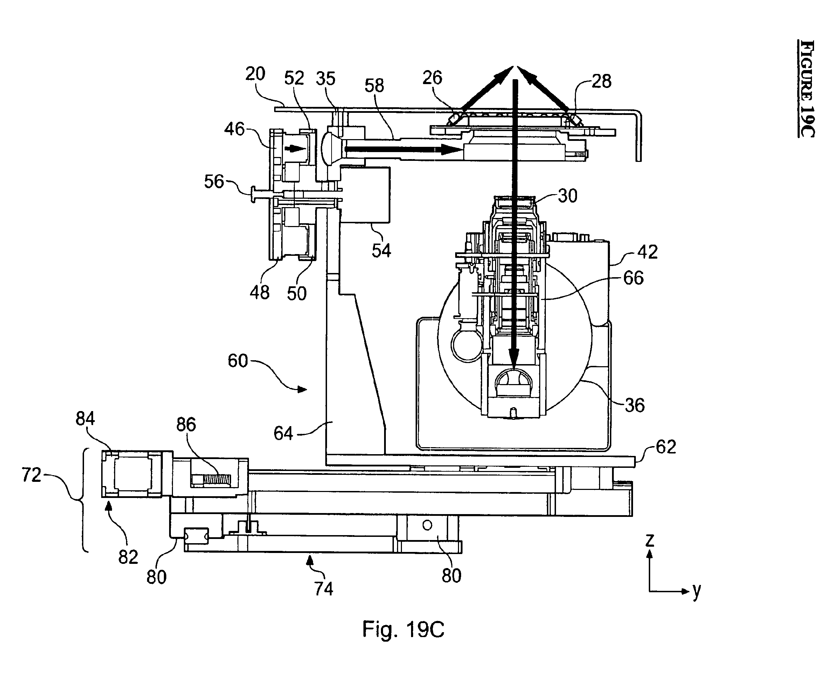

Figures 19A, 19B and 19C are perspective and orthogonal side views of the optics sub-assembly arranged below the main bed of the apparatus of Figure 16. These three figures are described together, rather than in turn, since they are different views of the same equipment, noting that not all features are visible or marked with reference numerals in each figure.

The previously described collar-mounted LED ring 24, 26, 28 is evident in all three figures. The LED collar 28 is cantilevered out on a side bracket from a vertical mounting plate 65 (Figure 19A) which is part of a frame 60. The vertical mounting plate 65 is upstanding from a base plate 62.

The fluorescence excitation optics is mounted on the base plate 62 via a further vertical mounting plate 64. The excitation source is colored LEDs 44 (not shown) that are arranged in groups of different colors 46 on a wheel 48 which is a converted filter wheel with LED groups 46 arranged at each filter position. In front of each LED group 46 there is a bandpass or other suitable narrowband filter 50 (see Figures 19B & 19C) each arranged in the filter position of a further filter wheel 52 arranged coaxially and on the same motor spindle 56 as the filter wheel 48, the two wheels being driven in unison by a motor 54. Each bandpass filter 50 is selected to transmit a range of wavelengths matched to the emission wavelength band of the LED group 46 with which it is paired. Light from the uppermost LED group 46 is directed horizontally through a light pipe 58, which is not a waveguide, merely a shroud for preventing light spillage, onto the semi-silvered mirror 32 (see Figure 19B and also Figure 17) which serves as a beam splitter for directing a portion of the colored LED light through the LED collar's aperture 25 to the object position. Other forms of beam splitter could also be used, for example a cubic beam splitter. The beamsplitter is preferably removable, or movable away from the aperture 25 so that when lateral illumination from the colored LED groups is not needed, it can be taken out of the collection path so that it does not result in loss of collected signal. A mounting stub 35 is also evident in Figures 19A and 19C. This mounting stub 35 is for connecting the colored LED group features to the top plate 20 (removed in Figure 19A, but shown in Figures 19B and 19C and also Figure 17).

The collection lens 30 is held vertically in a mounting tube 66 (see Figures 19B & 19C) at the base of which is arranged a plane deflecting mirror 68 which redirects the collected light horizontally and supplies it along a light pipe 70 to a CCD camera 34. Part way along the light pipe 70 there is arranged a filter wheel 36 mounted on a spindle 40 and driven by a motor 38. Drive electronics for the filter wheel 36 are housed in a unit 42. Typically filters will be used in the collection optics to filter out excitation light from the colored LED groups 46 when spectroscopic measurements are being performed. Collection side filters 45 may also be useful for filtering out fluorescence, e.g. to stop fluorescence from swamping out contrast of the cell periphery. This might be auto-fluorescence or fluorescence from a tag. For straightforward confluence detection using the white LEDs 24, no filter may be needed on the collection side.

The optical components are thus all mounted directly or indirectly on the base plate 62. The base plate 62 is carried by a linear positioner 82 which is in turn carried by a linear positioner 74 to provide xy-motion for the whole optical set-up. In the illustration, the x-positioner 74 is at the bottom with the y-positioner mounted on top of it. However, it will be appreciated this choice is arbitrary. It will also be appreciated that a parallel mechanism xy-positioner could be provided instead of two piggy-backed linear positioners. The x-positioner 74 comprises a motor 76, lead screw 78 and a pair of sets of guide bearings 80. The y-positioner 82 is the same, comprising a motor 84, lead screw 86 and a pair of sets of guide bearings 88.

As an alternative to having colored LED of different colors arranged in filter positions on a filter wheel as described above, it is possible to have concentric rings of different colors of LED in a single mounting. For example, the white light LED ring could be exchanged or supplemented with a number of LED rings of different colors. In principle an arbitrary arrangement of LEDs of different colors would provide the same functionality so long as LEDs of different colors could be driven independently, but would be a less elegant design. It would also be possible to use a single group of broadband LEDs in combination with filtering. However, this approach would tend to provide less illumination power than using different colors of LED. It will also be appreciated that other optical sources could be used including superfluorescent LEDs or diode lasers. Fixed wavelength or tunable diode lasers may be used.

By way of example, the table below gives, for a number of useful dyes, suitable LED types for the excitation LED groups 46 together with suitable pairs of excitation side filters 50 and collection-side (i.e. emission) filters 45. The peak excitation and emission wavelengths λ of the example dyes are also stated.

| Dye | Peak Excitation λ (nm) | Peak Emission λ (nm) | LED Type | Excitation Filter | Emission Filter (Chroma Co.) |
|---|---|---|---|---|---|
| | | | | | |
| BFP | 381 | 445 | UV | none | D460/50m |
| CFP | 434 | 477 | Royal Blue | D(HQ)450/50X | D505/40m |
| EGFP | 488 | 507 | Blue | D(HQ)470/40X | HQ535/50m |
| FITC | 490 | 525 | Blue | D(HQ)470/40X | HQ535/50m |
| YFP | 513 | 527 | Cyan | D(HQ)500/30X | D550/40m |
| Rhodamine | 550 | 573 | Green | D(HQ)530/30X | HQ590/50m |
| DSRed | 565 | 582 | Green | D(HQ)530/30X | HQ590/50m |
| Cy5 | 649 | 670 | Red | D(HQ)623/36X | HQ700/75m |

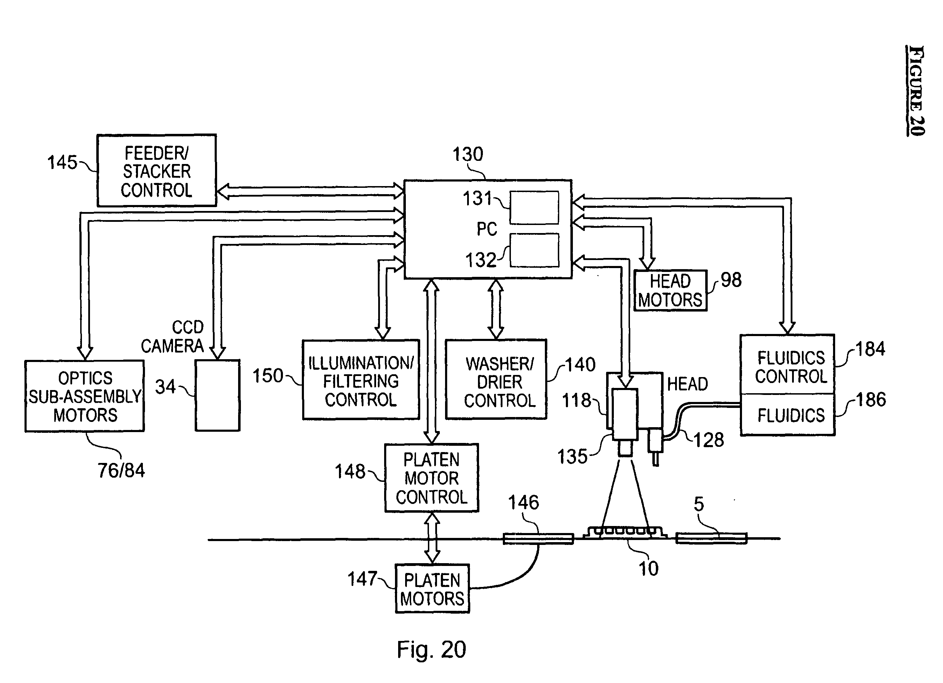

Figure 20 is a block schematic diagram showing the control system of the apparatus for coordinating the various components to perform the processes described above. A computer (PC 130) is used as the principal control component and is connected by electronic links using standard interfacing protocols to the various components that are part of the automated control system. The control is effected by control software 131 resident in the PC 130. Image processing and spectroscopic analysis software 132 is also resident in the PC 130 and linked to the control software 131. The image processing and spectroscopic analysis may also be carried out in hardware or firmware if desired. The CCD camera 34 is connected to the PC 130 for receiving digital images captured by the camera 34. An illumination and filter controller 150 is connected to the PC 130 for controlling the various under-bed optical sources and filter wheels of the optical sub-assembly 110. A washer/drier controller 140 is connected to the PC 130 and used to control the blower and the halogen lamps of the wash/dry station 102. The positioners 98 for moving the head 118 are connected to the PC 130. The PC 130 is also connected to the motors 76 and 84 of the x- and y-positioners of the under-bed optics sub-assembly 110. A head-mounted camera 135 is also provided for machine vision, such as bar-code detection on plates, and is connected to the PC 130 for receiving digital images captured by the head-mounted camera 135. These are used for aligning the pins of the head with the various locations of interest such as the wash/dry station 102, plates etc. The fluid lines 128 are connected to the fluidics unit 186 which is controlled by the fluidics control unit 184 connected to the PC 130. The fluidics control unit 184 is used to control the pressure in the fluid lines to allow aspiration, retention and expulsion of liquid from the sample. The fluidics control unit 184 also controls the wash cycle of the pins and fluid lines, whereby cleaning fluid from the baths is aspirated and expelled from the ends of the pins during the cleaning cycle. A feeder/stacker control unit 145 is also provided for the feeder/stacker units, including the plate supply lanes, and is connected to the PC 130. Separate units 145 may be provided for each lane in view of the modular nature of the feeder/stacker assemblies. The figure also illustrates schematically an optional feature whereby a carrier in the form of a platen 146 is provided to carry one or more plates 10 or other biological sample containers. The platen 146 is movable in the x- and y-directions by associated motors 147 and motor controller unit 148 which is connected to the PC 130, these elements collectively forming a positioning system for plates or other containers arranged on the apparatus. The platen can then be moved in a controlled fashion to allow iterative scanning by the optical system across all wells of a plate. The platen may be provided with an integral heating element, so that plates or other biological sample containers carried by the platen can be maintained at elevated temperatures, for example to promote enzymatic activity in the samples.

It will thus be appreciated that lateral positioning can be achieved in a variety of ways either by moving the optical source and detector on a common platform under the bed of the apparatus, moving the sample with its own xy-positioning system on the sample carrier, or by moving the head. In any given apparatus or process, various combinations of these motion systems may be used.

In summary, the described robotic apparatus has a sample manipulation head with associated positioning system mounted above the main bed of the apparatus, and can be used for picking of cells, in particular animal cells, or for other biological or chemical applications. An imaging station is arranged on the main bed where a sample container containing a sample can be placed in an object position. Both excitation and collection optical sub-systems are mounted under the main bed of the apparatus for performing spectroscopic analysis on a sample at the imaging station. The integration is based on a reflection mode optical solution, which allows all the optical components to be mounted under the main bed of the apparatus. Consequently, ancillary software driven or manual processes can be carried on with whether or not spectroscopic measurements are being made.

However, it will be appreciated that methods according to the invention can be performed on different apparatus than described herein. In particular, imaging tasks can be carried out in a conventional stand-alone imager, such as a Fuji LAS-1000, and picking tasks with a conventional picking robot, such as a Genetix QPix.

### EXAMPLES

### Example 1. General Protocol for Antigen-Based Detection of Antibody Secretion by Mammalian Cell Colonies

This Example illustrates a general method to selectively detect and isolate colonies producing a specific antibody by direct supplementation of methylcellulose-based medium with fluorescently-conjugated antigen

### Materials

Methylcellulose-based semi-solid medium or concentrate

Cell culture medium

FCS if appropriate

PSG (Pen-Strep-Gln) if appropriate

Unconjugated or rhodamine/phycoerythrin-conjugated goat anti human or anti mouse IgG or F(ab')₂ fragment (>1mg/ml)

FITC-conjugated antigen (>1mg/ml)

*Procedure*

Start with 40mls methylcellulose medium or concentrate. Add medium, FCS and other components to 90-100mls depending on protocol. Add 800µl PSG. Shake vigorously to mix.

Add anti-IgG to final concentration of 7.5µg/ml.

Add FITC-conjugated antigen to final concentration of 5.0µg/ml.

Invert gently but thoroughly to mix.

Add a sufficient number of cells to give ~50-100 colonies per well of a 6-well plate. For selection procedures (transfections or hybridoma fusions) the optimal number should be determined empirically. We suggest a figure of 25000-50000 cells/ml. For procedures without selection, add 400-1000 cells per ml.

Gently invert tube 10 times to distribute cells evenly. Leave to settle for 20 mins at 37°C.

Plate out 2 mls per well into 6-well plates using a 10-20ml disposable pipette. Tilt plate gently to ensure even distribution.

Incubate at 37°C in CO₂ incubator to allow colonies to grow. Do not disturb.

At day 3 and beyond, use ClonePix^{FL} to image, detect and isolate colonies accumulating fluorescence.

### Example 2. Antigen-Specific Detection of Antibody Secretion

NS0 cells stably expressing human IgG1 against a bacterial protein are grown into clonal colonies in methylcellulose-based semi-solid medium containing 7.5µg/ml unconjugated goat anti-human IgG and 5µg/ml FITC-conjugated bacterial protein antigen.

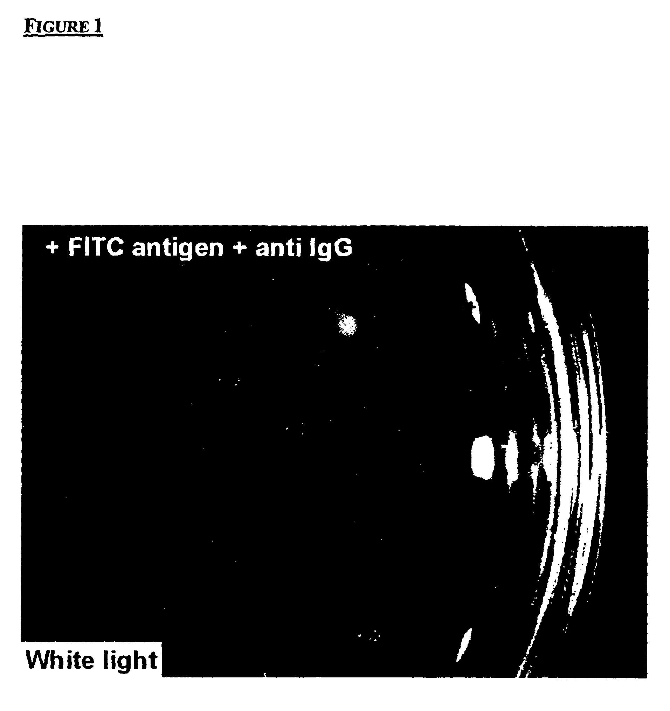

Figure 1 is a white light image captured at day 6 which shows many colonies.

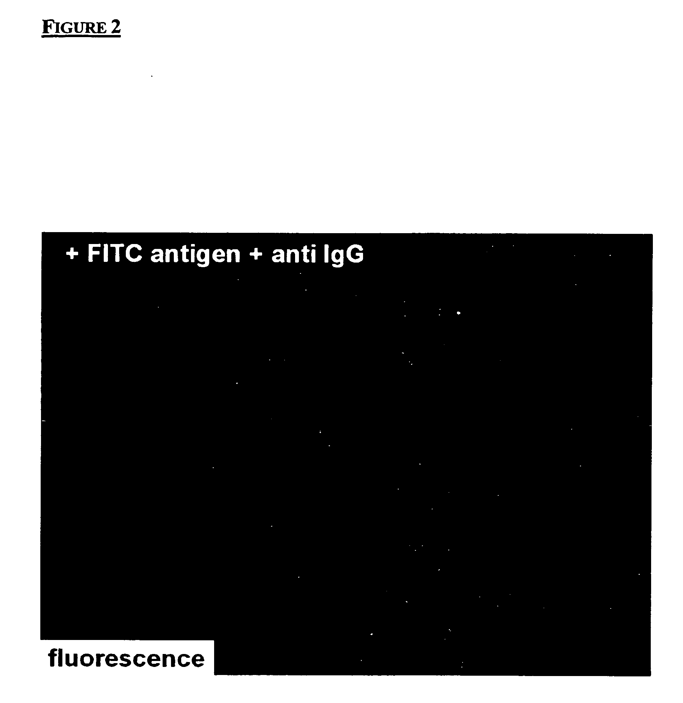

Figure 2 is a corresponding fluorescent image captured at day 6 which shows fluorescence accumulating around the few colonies secreting the human IgG1.

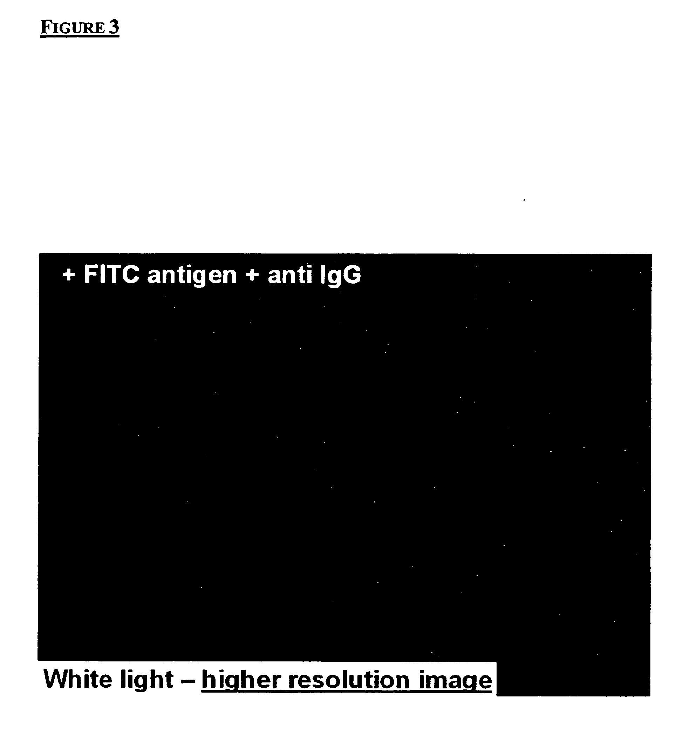

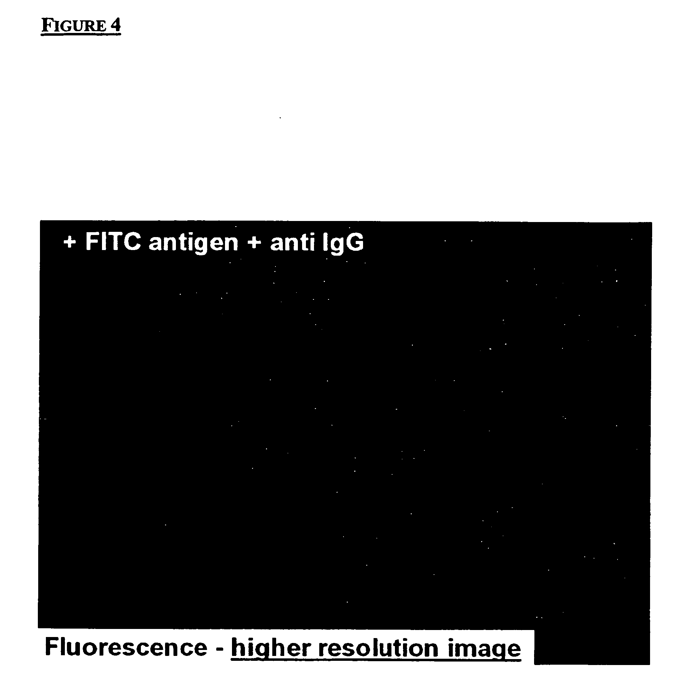

Figure 3 and Figure 4 show corresponding images as Figure 1 and Figure 2, but at higher resolution.

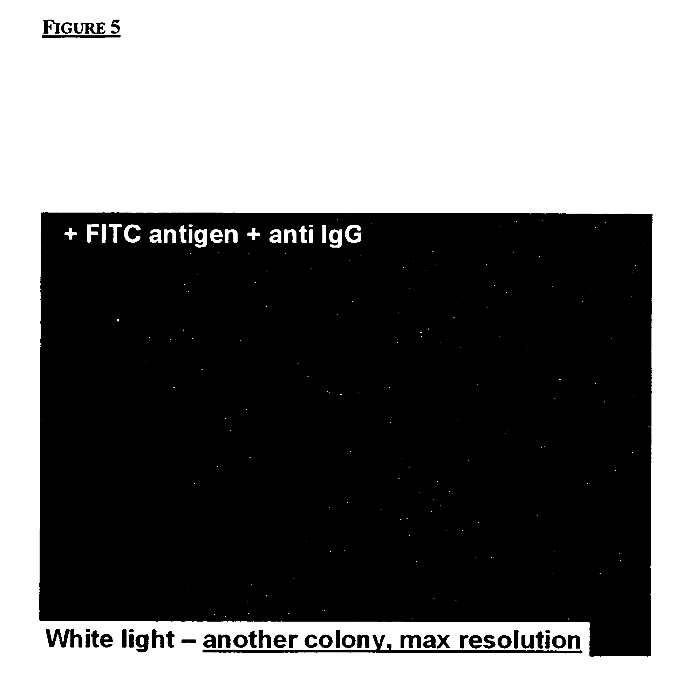

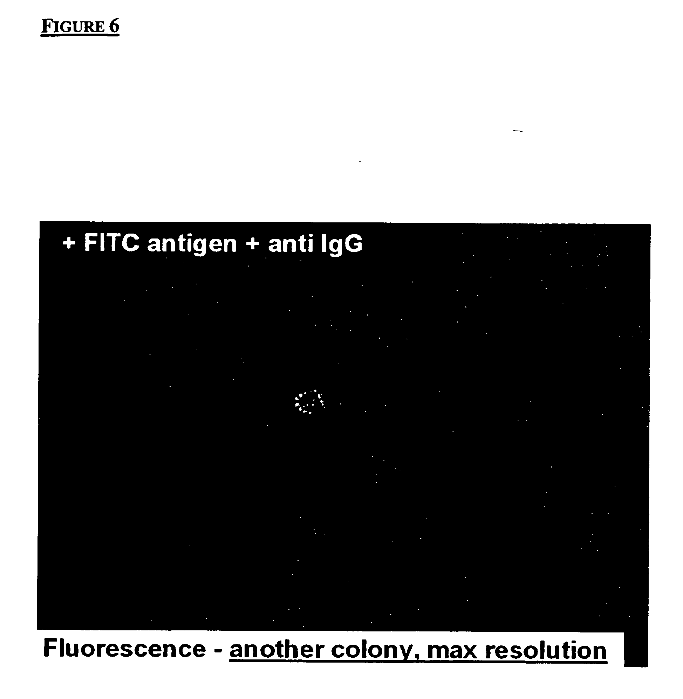

Figure 5 and Figure 6 show corresponding images as Figure 1 and Figure 2, but at maximum resolution of the ClonePixFL

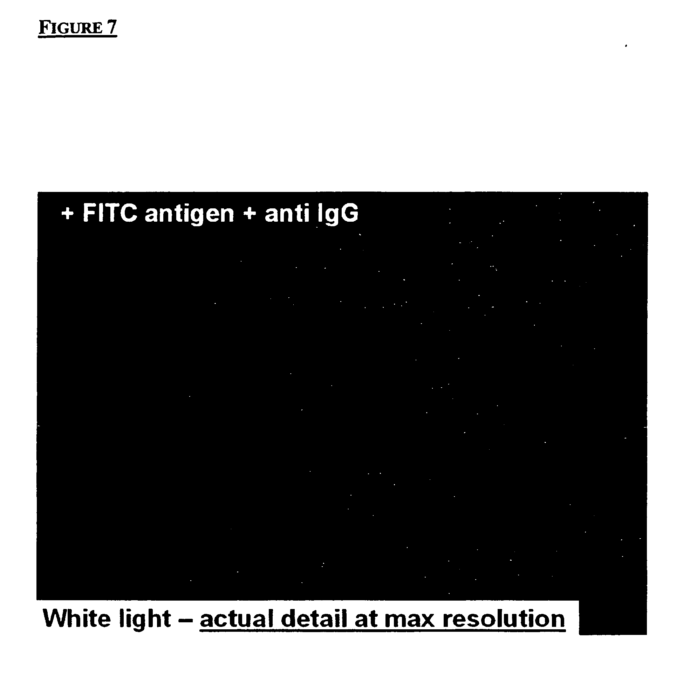

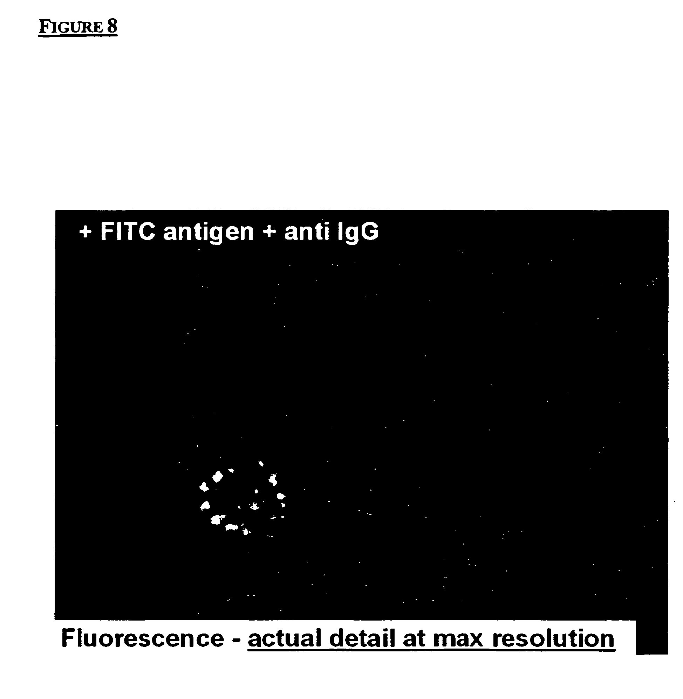

Figure 7 and Figure 8 correspond to Figure 5 and Figure 6, digitally zoomed on one region.

### Example 3. Antigen-Specific Detection of Antibody Secretion - Control Without Class Marker

This Example illustrates a control for Example 2, specifically the effect of omitting the unconjugated goat anti-human IgG.

NS0 cells stably expressing human IgG1 against a bacterial protein are grown into clonal colonies in methylcellulose-based semi-solid medium containing 5µg/ml FITC-conjugated bacterial protein antigen but no unconjugated goat anti-human IgG.

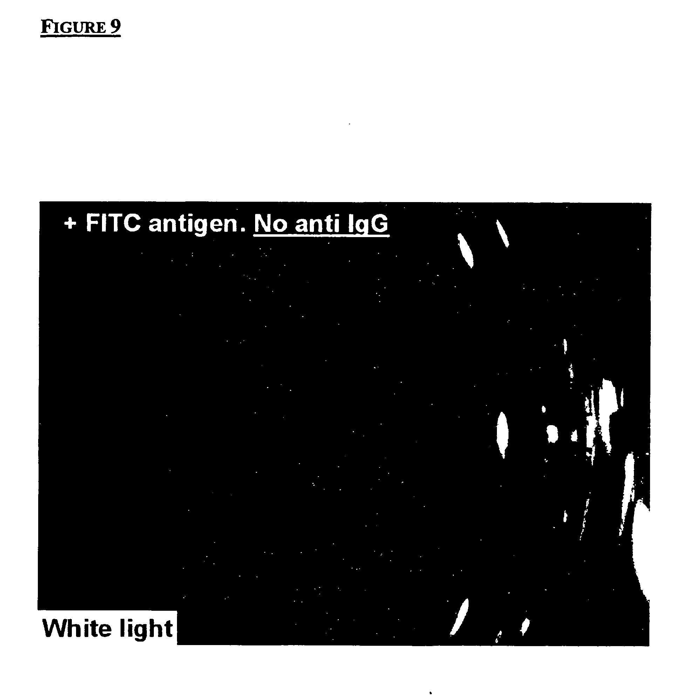

Figure 9 is a white light image of such a control captured at day 6 which shows many colonies.

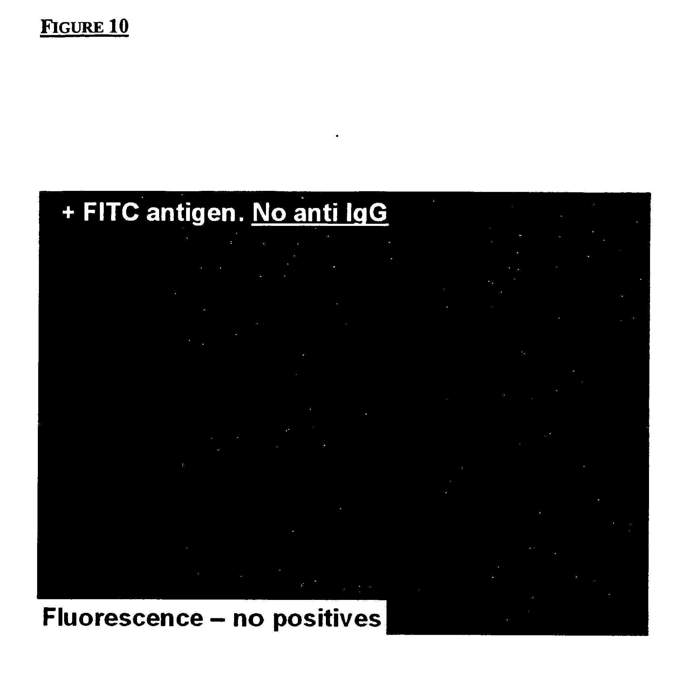

Figure 10 is a corresponding fluorescent image captured at day 6 which shows no positive colonies. The faint spots are due to cellular auto-fluorescence and simply mirror the white light image.

### Example 3. Antigen-Specific Detection of Antibody Secretion - Control Without Specificity Marker

This Example illustrates a control for the Example 2, specifically the effect of using a non-reacting bacterial protein in place of the specific antigen.

NS0 cells stably expressing human IgG1 against a bacterial protein are grown into clonal colonies in methylcellulose-based semi-solid medium containing 7.5µg/ml unconjugated goat anti-human IgG and 5µg/ml FITC-conjugated control bacterial protein antigen.

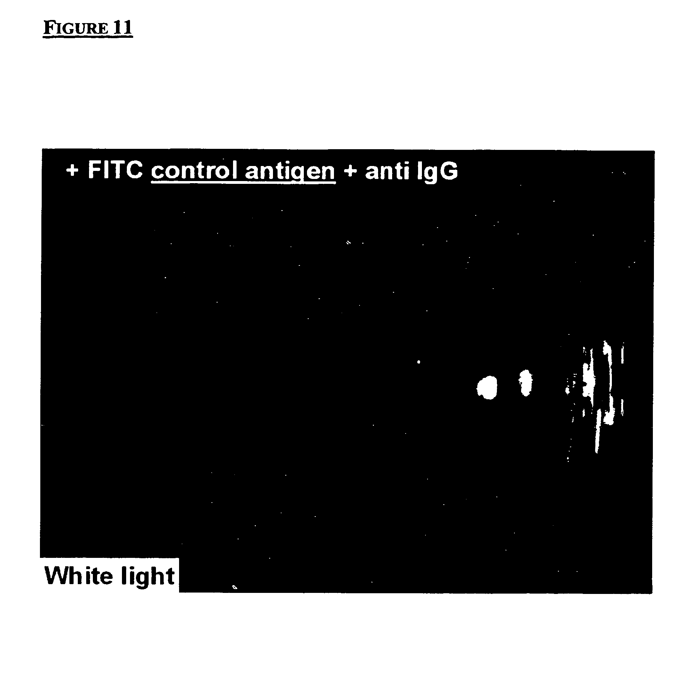

Figure 11 is a white light image of such a control captured at day 6 which shows many colonies.

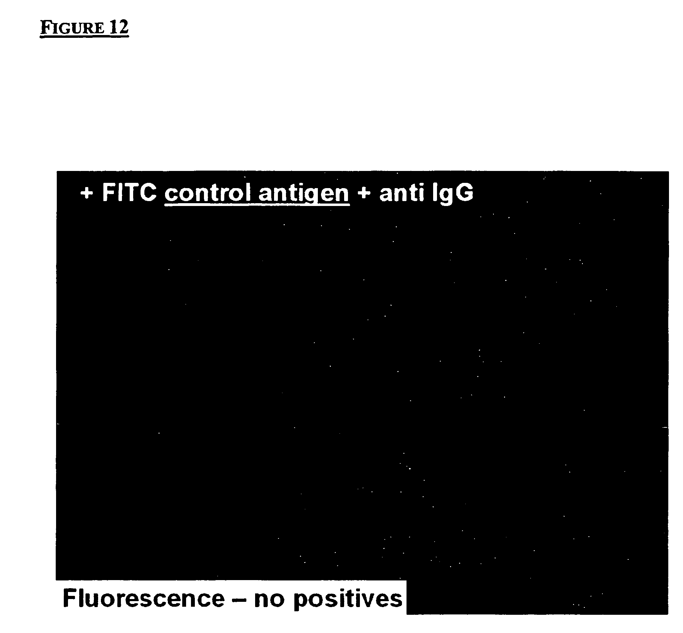

Figure 12 is a corresponding fluorescent image captured at day 6 which shows no positive colonies. The faint spots are due to cellular autofluorescence and simply mirror the white light image.

### Example 4. Relationship Between Fluorescence Secretion and Antibody Productivity

This Example examines whether fluorescence detection of secretion is a predictor of antibody productivity.

The IgG expressing NS0 line described above is plated out in the presence of FITC anti human IgG, and then high, medium and low fluorescing colonies are picked and IgG1 antibody production is measured directly.

Specifically, IgG1 expressing NS0 cells are grown into colonies for 7 days in semi-solid medium, and quantified for antibody production using FITC anti human IgG.

High, medium and low fluorescing colonies are picked to a 96-Well plate, grown on for 7days and transferred to 24-Well plates for 11 days until <30% viable. Supernatants are collected and antibody production measured using a Protein A / HPLC capture and quantitation method.

Figure 13A, Figure 13B and Figure 13C show the relationship between fluorescence detection and antibody productivity.

Figure 13A is a comparison of production in the three groups, and shows that productivity in the brightest fluorescent colonies is significantly greater than the medium and low fluorescent colonies.

Figure 13B shows a correlation of production with colony fluorescence intensity. The Figure demonstrates that there is a significant correlation between colony fluorescence and antibody productivity.

Figure 13C shows the population distribution of all colonies in the original sample (n=123) from which the high, medium and low fluorescing colonies are selected. Note that the brightest colonies (and highest antibody expressers) account for only a small proportion of the population.

We speculate that the two colonies that showed good fluorescence, but later show a low rate of antibody production, may have been unstable.

### Example 5. Automated Picking Using ClonePixFL Robot

This Example describes a general protocol for using a robotic apparatus comprising an imager and a picker to automate picking of cells or colonies expressing a polypeptide of interest.
(1) Plate cells into 6-Well culture plates. Add appropriate fluorescent probe to the semi-solid medium.
(2) Grow colonies for 3 to 12 days.
   [Steps (1) and (2) may be carried out as described above in Examples 1-4]
(3) Place plates in ClonePixFL.
(4) ClonePixFL automatically images plates.
(5) ClonePixFL detects colonies and secretion halos where applicable.
(6) ClonePixFL generates a highly selective pick list of colonies based on user-defined criteria.
(7) ClonePixFL automatically picks the selected clonal colonies and disperses them to 96-Well plates prefilled with appropriate medium.
(8) ClonePixFL automatically saves captured images and logs colony criteria and microplate location for export or LIMS management.
(9) Remove plates from ClonePixFL.
(10) Grow clonal cells in 96-Well plates.
(11) The 6-Well source plates can be re-entered for further rounds of picking.

Although the protocol refers specifically to ClonePixFL as an example of such a robotic apparatus, it will be clear to the skilled reader that any robotic apparatus capable of imaging colonies and picking selected ones may be used in the method. Such a robotic apparatus will generally comprise an imager for visualising colonies and enabling selection of colonies of interest (e.g., those colonies which are labelled) and a colony picker, to enable such colonies of interest to be picked. For example, a description of a robotic apparatus is provided in the section above "Robotic Detection and Picking"; with reference to that description, the colonies may be imaged using the imaging station 100, and picked using the cell-picking head 118.

### Example 6. Detection of Antibody Secreting Colonies and Class

This example illustrates the use of an indirectly labelled antigen to detect Hybridoma colonies secreting a specific antibody, combined with a fluorescently labelled anti-IgG to confirm the immunoglobulin class. Colonies positive for both parameters are isolated.

A Hybridoma fusion is recovered and plated into methylcellulose based semi-solid medium as described in Example 1. The methylcellulose is directly supplemented with the detection materials listed below.

Detection Materials: Biotinylated antigen at a concentration of 5µg/ml; FITC-Streptavidin at a concentration of 2.5µg/ml; AlexaFluor546 conjugated goat anti-mouse IgG at a concentration of 7.5µg/ml.

The fusion is grown in the presence of HAT selection in the semi-solid media for 7 days.

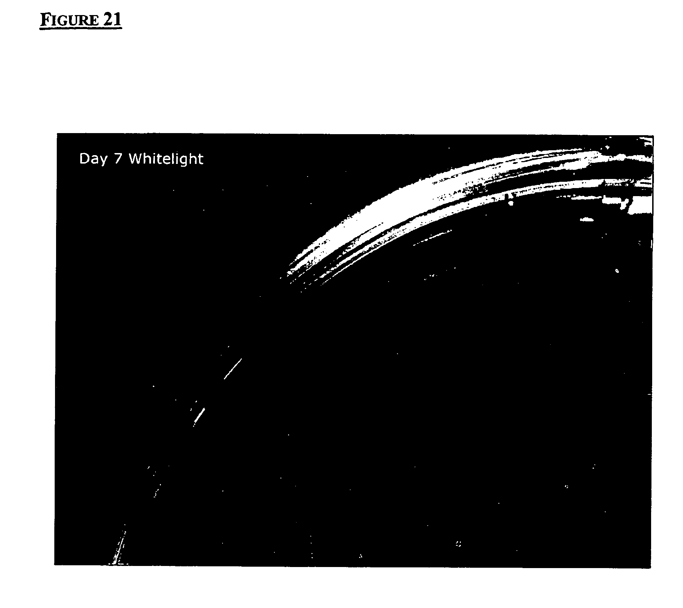

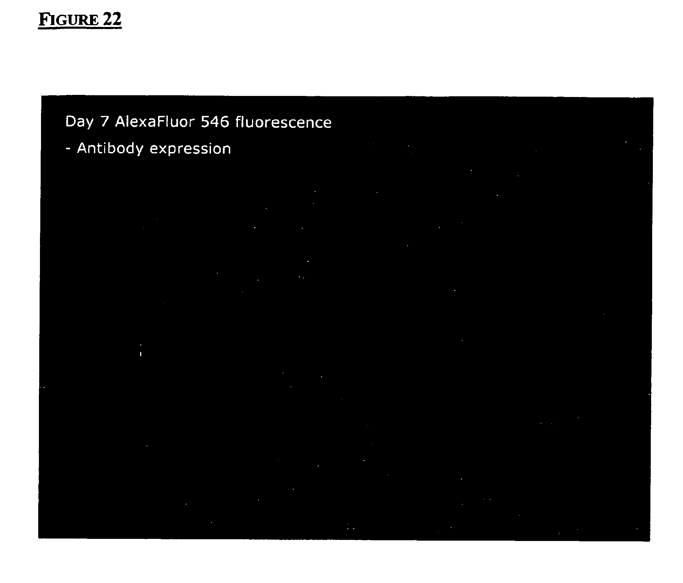

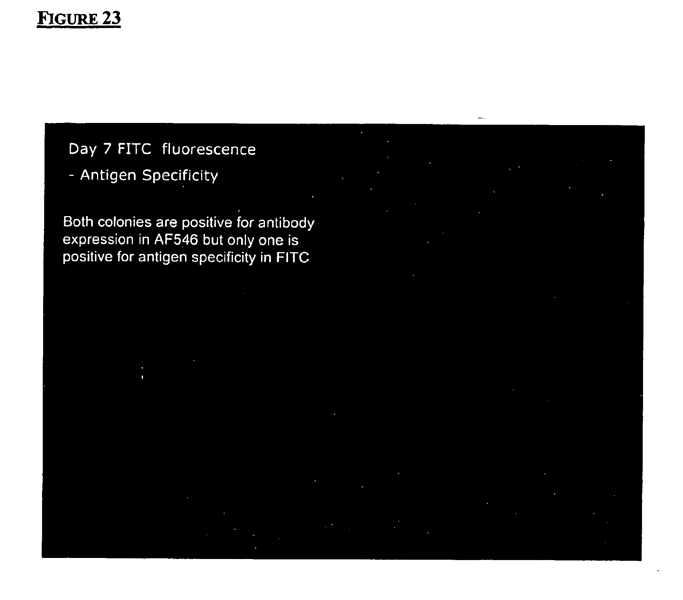

Figure 21 is an image taken on the ClonePixFl showing two colonies in white light. Figure 22 shows the same two colonies in AlexaFluor 546 fluorescence, demonstrating that both colonies are secreting an IgG . Figure 23 shows the same two colonies in FITC fluorescence, demonstrating that only one of the two colonies is positive for the antigen.

Using both of these parameters to detect colonies allows a further selection measure in that colonies must be secreting an antibody which is both specific to the antigen and of the correct immunoglobulin class e.g. IgG and not IgM. It also allows a measure of the specificity of the antibody to the antigen. A colony which is secreting a large amount of antibody but binding little antigen is less desirable than a colony secreting slightly lower antibody but binding the antigen better.

Various modifications and variations of the described methods and system of the invention will be apparent to those skilled in the art. Indeed, various modifications of the described modes for carrying out the invention which are obvious to those skilled in molecular biology or related fields may be made. Furthermore, various combinations of the features of the following dependent claims can be made with the features of the independent claims.

## Claims

1. A method of detecting a polypeptide of interest produced by a cell or a cell colony, the method comprising:
(a) exposing the polypeptide to (i) a class marker capable of identifying a class of polypeptides to which the polypeptide of interest belongs; and (ii) a specificity marker capable of identifying a specific binding ability of the polypeptide of interest; and
(b) detecting binding of the polypeptide to the class marker and the specificity marker,
wherein the class marker-polypeptide complex is associated with, coincident with or around the cell or the cell colony and wherein the size or extent of the complex is detected to provide a measure of the amount of polypeptide produced by the cell or cell colony, and wherein detecting binding to the class marker and the specificity marker is done simultaneously or in sequence.

2. The method according to Claim 1, in which the class marker comprises a polyclonal antibody capable of binding to polypeptides of a class to which the polypeptide of interest belongs.

3. The method according to Claim 1 or 2, in which the specificity marker comprises a binding partner capable of specifically binding to the polypeptide of interest.

4. The method according to Claim 1, 2 or 3, in which the cell or cell colony is disposed on or in solid or semi-solid media, preferably methylcellulose media.

5. The method according to any preceding claim, in which the polypeptide is secreted by the cell.

6. The method according to any preceding claim, in which the class marker-polypeptide complex forms a halo or aura around the cell or cell colony.

7. The method according to any preceding claim, in which binding between the specificity marker and the polypeptide is detected to provide a measure of the binding specificity of the polypeptide of interest.

8. The method according to any preceding claim, in which binding between the polypeptide and the class marker and binding between the polypeptide and the specificity marker is detected simultaneously.

9. The method according to any preceding claim, in which the class marker comprises a small molecule, preferably a synthetic small molecule.

10. The method according to any preceding claim, in which the class marker, or the specificity marker, or both, is labelled with a reporter, preferably a fluorescent molecule, preferably a fluorophore, more preferably a fluorochrome.

11. The method according to any preceding claim, in which the class marker and the specificity marker are labelled with two different fluorophores emitting fluorescent light of different wavelengths.

12. The method according to Claim 10 or 11, in which the fluorophores comprise fluorescein, tetramethylrhodamine or phycoerythrin.

13. The method according to any preceding claim, in which the polypeptide of interest comprises a recombinant polypeptide, preferably expressed by a transfected cell such as a suspension adapted cell-line.

14. The method according to any preceding claim, in which the cell or cell colony is a hybridoma cell or cell colony, including a transfected hybridoma, and the polypeptide of interest comprises an immunoglobulin or antibody.

15. The method according to Claim 14, in which the class of polypeptides is an Ig class, preferably an IgG class.

16. The method according to Claim 14 or 15, in which the class marker comprises a polyclonal anti-IgG antibody.

17. The method according to Claim 14, 15 or 16 in which the specificity marker comprises an antigen or epitope of the immunoglobulin or antibody.

18. The method according to any preceding claim, in which the polypeptide of interest comprises a receptor, preferably a cell surface receptor.

19. The method according to Claim 18, in which the class of polypeptides is a receptor subclass, preferably a seven transmembrane (7TM) receptor subclass.

20. The method according to Claim 18 or 19, in which the class marker comprises an antibody capable of binding to a receptor subclass, preferably an antibody capable of binding to a seven transmembrane (7TM) receptor subclass.

21. The method according to Claim 18, 19 or 20 in which the specificity marker comprises a ligand, preferably an affinity ligand, of the receptor.

22. A method of identifying the cell type of a cell or colony, the method comprising detecting whether the cell or colony produces a polypeptide known to be associated with a particular cell type by a method according to any preceding claim.

23. A method of selecting a cell or colony of a cell type of interest from a plurality of cells or colonies, the method comprising detecting whether a candidate cell or colony produces a polypeptide known to be associated with the cell type of interest by a method according to any of Claims 1 to 21, and optionally picking the selected cell or colony.

24. The method according to any of Claims 1 to 21 for assessing the productivity of a hybridoma cell or cell colony in producing an antibody of interest, and optionally, the specificity of said antibody.

25. A method of selecting a productive cell or cell colony from a plurality of cells or cell colonies, the method comprising:
(a) assessing the amount of polypeptide of interest produced by a relevant cell or cell colony by a method according to any of Claims 1 to 21;
(b) comparing said amount to a predetermined amount;
(c) selecting the cell or cell colony if it meets or exceeds the predetermined amount.

26. The method according to Claim 25 for selecting a set of productive cells or cell colonies which meet or exceed the predetermined amount.

27. The method according to Claim 25 or 26 for detecting a specificity of a polypeptide produced by the cell or cell colony, which further comprises the steps of:
(a) assessing the binding specificity of polypeptide of interest produced by the cell or cell colony by a method according to any of Claims 1 to 21;
(b) selecting the cell or cell colony if it produces a polypeptide which binds specifically to a binding partner of interest.

28. The method according to any preceding claim, in which the plurality of cells or cell colonies is disposed on a planar substrate such as a culture dish or plate.

29. A method of picking a cell or cell colony productive for a polypeptide, the method comprising assessing the amount of polypeptide of interest produced by the cell or cell colony by method according to any of Claims 1 to 21, and picking that cell or cell colony.

30. The method according to any preceding claim, in which the class marker binds to and aggregates the polypeptide to enable detection of a signal from the label on the specificity marker.

31. Use of a robot equipped with a head for manipulating biological samples, the use comprising:
providing a sample container containing at least one cell capable of producing a polypeptide of interest, wherein the polypeptide has been exposed: (a) to a class marker capable of identifying a class of polypeptides to which the polypeptide of interest belongs; and (b) to a specificity marker capable of identifying a specific binding ability of the polypeptide of interest;
arranging the sample container at an imaging station;
making a spectroscopic measurement by illuminating the at least one cell from below and collecting light from the at least one cell also from below in order to detect binding of the polypeptide to the class marker or the specificity marker or both, wherein the class marker-polypeptide complex is associated with, coincident with or around the cell or the cell colony and wherein the size or extent of the complex is detected to provide a measure of the amount of polypeptide produced by the cell or cell colony; and
manipulating the at least one cell with the head based on the spectroscopic measurement of the binding of the polypeptide.

32. The use of claim 31, wherein the spectroscopic measurement is based on a fluorescent agent.

33. The use of claim 32, wherein the fluorescent agent is a dye.

34. The use of claim 33, wherein the dye is contained in, on or around the at least one cell.

35. The use of claim 33, wherein the at least one cell is contained in a medium which is marked with the dye whose optical activity is modified by secretion from the at least one cell.

36. The use of claim 35, wherein the spectroscopic measurement detects fluorescence, white light, infra-red, by Raman spectroscopy, or auto-fluorescence.

37. The use of claim 32, wherein the fluorescent agent is an inherent part of the cell.

38. The use of claim 31, wherein the at least one cell is an animal cell.

39. The use of claim 32, wherein the fluorescent agent is detected by white light or infra-red.

40. The use of claim 31, wherein the spectroscopic measurement is based on a Raman agent.

## Patentansprüche

1. Verfahren zum Detektieren eines durch eine Zelle oder eine Zellkolonie produzierten interessierenden Polypeptids, wobei das Verfahren folgendes umfaßt:
(a) Aussetzen des Polypeptids an (i) einen Klassenmarker, der eine Klasse von Polypeptiden, zu welcher das interessierende Polypeptid gehört, identifizieren kann, und (ii) einen Spezifitätsmarker, der eine spezifische Bindungsfähigkeit des interessierenden Polypeptids identifizieren kann, und
(b) Detektieren einer Bindung des Polypeptids an den Klassenmarker und den Spezifitätsmarker,
wobei der Klassenmarker-Polypeptid-Komplex verknüpft ist mit oder deckungsgleich ist mit der Zelle oder der Zellkolonie oder um diese herum und wobei die Größe oder der Umfang des Komplexes bestimmt wird, um ein Maß für die Menge an Polypeptid, das durch die Zelle oder Zellkolonie erzeugt wird, bereitzustellen, und wobei das Detektieren von Bindung an den Klassenmarker und den Spezifitätsmarker gleichzeitig oder nacheinander durchgeführt wird.

2. Verfahren nach Anspruch 1, wobei der Klassenmarker einen polyklonalen Antikörper umfaßt, der in der Lage ist, an Polypeptide einer Klasse, zu welcher das interessierende Polypeptid gehört, zu binden.

3. Verfahren nach Anspruch 1 oder 2, wobei der Spezifitätsmarker einen Bindungspartner umfaßt, der in der Lage ist, spezifisch an das interessierende Polypeptid zu binden.

4. Verfahren nach Anspruch 1, 2 oder 3, wobei die Zelle oder die Zellkolonie auf oder in festem oder halbfestem Medium, vorzugsweise Methylzellulosemedium, angeordnet ist.

5. Verfahren nach einem der vorangegangenen Ansprüche, wobei das Polypeptid von der Zelle abgesondert wird.

6. Verfahren nach einem der vorangegangenen Ansprüche, wobei der Klassenmarker-Polypeptid-Komplex einen Halo oder eine Aura um die Zelle oder Zellkolonie herum bildet.

7. Verfahren nach einem der vorangegangenen Ansprüche, wobei die Bindung zwischen dem Spezifitätsmarker und dem Polypeptid detektiert wird, um ein Maß für die Bindungsspezifität des interessierenden Polypeptids bereitzustellen.

8. Verfahren nach einem der vorangegangenen Ansprüche, wobei die Bindung zwischen dem Polypeptid und dem Klassenmarker und die Bindung zwischen dem Polypeptid und dem Spezifitätsmarker gleichzeitig detektiert werden.

9. Verfahren nach einem der vorangegangenen Ansprüche, wobei der Klassenmarker ein kleines Molekül, vorzugsweise ein synthetisches kleines Molekül umfaßt.

10. Verfahren nach einem der vorangegangenen Ansprüche, wobei der Klassenmarker oder der Spezifitätsmarker oder beide mit einem Reporter, vorzugsweise einem fluoreszierenden Molekül, bevorzugt einem Fluorophor, bevorzugter einem Fluorochrom, markiert ist bzw. sind.

11. Verfahren nach einem der vorangegangenen Ansprüche, wobei der Klassenmarker und der Spezifitätsmarker mit zwei verschiedenen Fluorophoren markiert sind, die fluoreszierendes Licht unterschiedlicher Wellenlängen emittieren.

12. Verfahren nach Anspruch 10 oder 11, wobei die Fluorophore Fluorescein, Tetramethylrhodamin oder Phycoerythrin umfassen.

13. Verfahren nach einem der vorangegangenen Ansprüche, wobei das interessierende Polypeptid ein rekombinantes Polypeptid umfaßt, welches vorzugsweise durch eine transfizierte Zelle, wie z.B. eine angepaßte Suspensionszellinie, exprimiert wird.

14. Verfahren nach einem der vorangegangenen Ansprüche, wobei die Zelle oder die Zellkolonie eine Hybridomzelle oder -zellkolonie, einschließlich eines transfizierten Hybridoms, ist und das interessierende Polypeptid ein Immunglobulin oder einen Antikörper umfaßt.

15. Verfahren nach Anspruch 14, wobei die Klasse von Polypeptiden eine Ig-Klasse, bevorzugt eine IgG-Klasse ist.

16. Verfahren nach Anspruch 14 oder 15, wobei der Klassenmarker einen polyklonalen Anti-IgG-Antikörper umfaßt.

17. Verfahren nach Anspruch 14, 15 oder 16, wobei der Spezifitätsmarker ein Antigen oder ein Epitop des Immunglobulins oder des Antikörpers umfaßt.

18. Verfahren nach einem der vorangegangenen Ansprüche, wobei das interessierende Polypeptid einen Rezeptor, vorzugsweise einen Zelloberflächenrezeptor, umfaßt.

19. Verfahren nach Anspruch 18, wobei die Klasse von Polypeptiden eine Rezeptor-Unterklasse, vorzugsweise eine Sieben-Transmembran- (7TM-) Rezeptor-Unterklasse ist.

20. Verfahren nach Anspruch 18 oder 19, wobei der Klassenmarker einen Antikörper, der in der Lage ist, an eine Rezeptor-Unterklasse zu binden, bevorzugt einen Antikörper, der zur Bindung an eine Sieben-Transmembran- (7TM-) Rezeptor-Unterklasse in der Lage ist, umfaßt.

21. Verfahren nach Anspruch 18, 19 oder 20, wobei der Spezifitätsmarker einen Liganden, vorzugsweise einen Affinitätsliganden des Rezeptors umfaßt.

22. Verfahren zum Identifizieren des Zelltyps einer Zelle oder Kolonie, wobei das Verfahren umfaßt, daß mittels eines Verfahrens nach einem der vorangegangenen Ansprüche detektiert wird, ob die Zelle oder die Kolonie ein Polypeptid produziert, von dem bekannt ist, daß es mit einem bestimmen Zelltyp assoziiert ist.

23. Verfahren zum Auswählen einer Zelle oder Kolonie eines interessierenden Zelltyps aus einer Mehrzahl von Zellen oder Kolonien, wobei das Verfahren umfaßt, daß man mittels eines Verfahrens nach einem der Ansprüche 1 bis 21 detektiert, ob eine Kandidatenzelle oder -kolonie ein Polypeptid produziert, von dem bekannt ist, daß es mit dem interessierenden Zelltyp assoziiert ist, und optional die ausgewählte Zelle oder Kolonie aufnimmt.

24. Verfahren nach einem der Ansprüche 1 bis 21 zum Bestimmen der Produktivität einer Hybridomzelle oder -zellkolonie bei der Herstellung eines interessierenden Antikörpers und optional der Spezifität des Antikörpers.

25. Verfahren zum Auswählen einer produktiven Zelle oder Zellkolonie aus einer Mehrzahl von Zellen oder Zellkolonien, wobei das Verfahren folgendes umfaßt:
(a) Bestimmen der Menge an interessierendem Polypeptid, welches durch eine relevante Zelle oder Zellkolonie produziert wurde, durch ein Verfahren nach einem der Ansprüche 1 bis 21,
(b) Vergleichen der Menge mit einer vorbestimmten Menge,
(c) Auswählen der Zelle oder der Zellkolonie, wenn sie die vorbestimmte Menge erreicht oder überschreitet.

26. Verfahren nach Anspruch 25 zum Auswählen eines Satzes produktiver Zellen oder Zellkolonien, die die vorbestimmte Menge erreichen oder überschreiten.

27. Verfahren nach Anspruch 25 oder 26 zum Detektieren einer Spezifität eines durch die Zelle oder Zellkolonie produzierten Polypeptids, wobei das Verfahren weiterhin die folgenden Stufen umfaßt:
(a) Bestimmen der Bindungsspezifität des interessierenden, von der Zelle oder der Zellkolonie produzierten Polypeptids durch ein Verfahren nach einem der Ansprüche 1 bis 21,
(b) Auswählen der Zelle oder der Zellkolonie, wenn sie ein Polypeptid produziert, welches spezifisch an einen interessierenden Bindungspartner bindet.

28. Verfahren nach einem der vorangegangenen Ansprüche, wobei die Mehrzahl von Zellen oder Zellkolonien auf einem ebenen Substrat, wie einer Kulturschale oder -platte, angeordnet ist.

29. Verfahren zum Aufnehmen einer Zelle oder einer Zellkolonie, die ein Polypeptid produziert, wobei das Verfahren umfaßt, daß man die Menge des von der Zelle oder der Zellkolonie produzierten interessierenden Polypeptids unter Verwendung des Verfahrens nach einem der Ansprüche 1 bis 21 bestimmt und diese Zelle oder Zellkolonie aufnimmt.

30. Verfahren nach einem der vorangegangenen Ansprüche, wobei der Klassenmarker an das Polypeptid bindet und mit diesem aggregiert, um die Detektion eines Signals von der Markierung an dem Spezifitätsmarker zu ermöglichen.

31. Verwendung eines Roboters, der mit einem Kopf für die Manipulation von biologischen Proben ausgerüstet ist, wobei die Verwendung folgendes umfaßt:
Bereitstellen eines Probenbehälters, welcher wenigstens eine Zelle enthält, die ein interessierendes Polypeptid produzieren kann, wobei das Polypeptid (a) einem Klassenmarker, der eine Klasse von Polypeptiden, zu der das interessierende Polypeptid gehört, identifizieren kann, und (b) einem Spezifitätsmarker, der eine spezifische Bindungsfähigkeit des interessierenden Polypeptids identifizieren kann, ausgesetzt wurde,
Anordnen des Probenbehälters an einer Abbildungsstation,
Durchführen einer spektroskopischen Messung durch Beleuchten der wenigstens einen Zelle von unten und Sammeln von Licht von der wenigstens einen Zelle ebenfalls von unten, um die Bindung des Polypeptids an den Klassenmarker oder den Spezifitätsmarker oder beide zu detektieren, wobei der Klassenmarker-Polypeptid-Komplex verknüpft ist mit oder deckungsgleich ist mit der Zelle oder der Zellkolonie oder um diese herum und wobei die Größe oder der Umfang des Komplexes bestimmt wird, um ein Maß für die Menge an Polypeptid, das durch die Zelle oder Zellkolonie erzeugt wird, bereitzustellen, und
Manipulieren der wenigstens einen Zelle mit dem Kopf auf Basis der spektroskopischen Messung der Bindung des Polypeptids.

32. Verwendung nach Anspruch 31, wobei die spektroskopische Messung auf einem fluoreszierenden Mittel basiert.

33. Verwendung nach Anspruch 32, wobei das fluoreszierende Mittel ein Farbstoff ist.

34. Verwendung nach Anspruch 33, wobei der Farbstoff in, auf oder um die wenigstens eine Zelle herum enthalten ist.

35. Verwendung nach Anspruch 33, wobei die wenigstens eine Zelle in einem Medium enthalten ist, welches mit dem Farbstoff markiert ist, dessen optische Aktivität durch Absonderung aus der wenigstens einen Zelle modifiziert wird.

36. Verwendung nach Anspruch 35, wobei die spektroskopische Messung Fluoreszenz, Weißlicht, Infrarot, mittels Raman-Spektroskopie oder Autofluoreszenz detektiert.

37. Verwendung nach Anspruch 32, wobei das fluoreszierende Mittel ein inhärenter Teil der Zelle ist.

38. Verwendung nach Anspruch 31, wobei die wenigstens eine Zelle eine Tierzelle ist.

39. Verwendung nach Anspruch 32, wobei das fluoreszierende Mittel mittels Weißlicht oder Infrarotlicht detektiert wird.

40. Verwendung nach Anspruch 31, wobei die spektroskopische Messung auf einem Raman-Mittel basiert.

## Revendications

1. Méthode pour détecter un polypeptide intéressant produit par une cellule ou une colonie de cellules, méthode comprenant :
(a) l'exposition du polypeptide à (i) un marqueur de classe capable d'identifier une classe de polypeptides auxquels le polypeptide intéressant appartient ; et (ii) un marqueur de spécificité capable d'identifier une capacité de liaison spécifique du polypeptide intéressant ; et
(b) la détection de la liaison du polypeptide au marqueur de classe et au marqueur de spécificité,
dans laquelle le complexe marqueur de classe-polypeptide est associé à, coïncidant avec, ou autour de, la cellule ou la colonie de cellules et dans laquelle les dimensions ou l'étendue du complexe sont détectées pour fournir une mesure de la quantité de polypeptide produite par la cellule ou la colonie de cellules, et dans laquelle la détection de la liaison marqueur de classe et au marqueur de spécificité est effectuée simultanément ou en séquence.

2. Méthode suivant la revendication 1, dans laquelle le marqueur de classe comprend un anticorps polyclonal capable de se lier aux polypeptides d'une classe à laquelle le polypeptide intéressant appartient.

3. Méthode suivant la revendication 1 ou 2, dans laquelle le marqueur de spécificité comprend un partenaire de liaison capable de se lier spécifiquement au polypeptide intéressant.

4. Méthode suivant la revendication 1, 2 ou 3, dans laquelle la cellule ou la colonie de cellules est disposée sur ou dans un milieu solide ou semi-solide, de préférence un milieu à base de méthylcellulose.

5. Méthode suivant l'une quelconque des revendications précédentes, dans laquelle le polypeptide est sécrété par la cellule.

6. Méthode suivant l'une quelconque des revendications précédentes, dans laquelle le complexe marqueur de classe-polypeptide forme un halo ou une auréole autour de la cellule ou de la colonie de cellules.

7. Méthode suivant l'une quelconque des revendications précédentes, dans laquelle la liaison entre le marqueur de spécificité et le polypeptide est détectée pour fournir une mesure de la spécificité de liaison du polypeptide intéressant.

8. Méthode suivant l'une quelconque des revendications précédentes, dans laquelle la liaison entre le polypeptide et le marqueur de classe et la liaison entre le polypeptide et le marqueur de spécificité sont détectées simultanément.

9. Méthode suivant l'une quelconque des revendications précédentes, dans laquelle le marqueur de classe comprend une petite molécule, de préférence une petite molécule synthétique.

10. Méthode suivant l'une quelconque des revendications précédentes, dans laquelle le marqueur de classe, ou le marqueur de spécificité, ou bien les deux marqueurs, sont marqués avec un rapporteur, de préférence une molécule fluorescente, de préférence un fluorophore, notamment un fluorochrome.

11. Méthode suivant l'une quelconque des revendications précédentes, dans laquelle le marqueur de classe et le marqueur de spécificité sont marqués avec deux fluorophores différents émettant de la lumière fluorescente à des longueurs d'ondes différentes.

12. Méthode suivant la revendication 10 ou 11, dans laquelle les fluorophores comprennent la fluorescéine, la tétraméthylrhodamine ou la phycoérythrine.

13. Méthode suivant l'une quelconque des revendications précédentes, dans laquelle le polypeptide intéressant comprend un polypeptide recombinant, exprimé de préférence par une cellule transfectée telle qu'une lignée cellulaire adaptée à la croissance en suspension.

14. Méthode suivant l'une quelconque des revendications précédentes, dans laquelle la cellule ou la colonie de cellules est une cellule d'hybridome ou une colonie de cellules d'hybridome, comprenant un hybridome transfecté, et le polypeptide intéressant comprend une immunoglobuline ou un anticorps.

15. Méthode suivant la revendication 14, dans laquelle la classe de polypeptides est une classe de Ig, de préférence une classe de IgG.

16. Méthode suivant la revendication 14 ou 15, dans laquelle le marqueur de classe comprend un anticorps polyclonal anti-IgG.

17. Méthode suivant la revendication 14, 15 ou 16, dans laquelle le marqueur de spécificité comprend un antigène ou épitope de l'immunoglobuline ou de l'anticorps.

18. Méthode suivant l'une quelconque des revendications précédentes, dans laquelle le polypeptide intéressant comprend un récepteur, de préférence un récepteur de surface cellulaire.

19. Méthode suivant la revendication 18, dans laquelle la classe de polypeptide est une sous-classe de récepteurs, de préférence une sous-classe de récepteurs à sept domaines transmembranaires (7TM).

20. Méthode suivant la revendication 18 ou 19, dans laquelle le marqueur de classe comprend un anticorps capable de se lier à une sous-classe de récepteurs, de préférence un anticorps capable de se lier à une sous-classe de récepteurs à sept domaines transmembranaires (7TM).

21. Méthode suivant la revendication 18, 19 ou 20, dans laquelle le marqueur de spécificité comprend un ligand, de préférence un ligand d'affinité, du récepteur.

22. Méthode pour identifier le type cellulaire d'une cellule ou colonie, méthode comprenant l'étape consistant à détecter si la cellule ou la colonie produit un polypeptide connu en tant que polypeptide associé à un type cellulaire particulier par une méthode suivant l'une quelconque des revendications précédentes.

23. Méthode pour sélectionner une cellule ou colonie d'un type cellulaire intéressant à partir d'une pluralité de cellules ou de colonies, méthode comprenant l'étape consistant à détecter si une cellule ou colonie candidate produit un polypeptide connu en tant que polypeptide associé au type cellulaire intéressant par une méthode suivant l'une quelconque des revendications 1 à 21, et facultativement le prélèvement de la cellule ou colonie sélectionnée.

24. Méthode suivant l'une quelconque des revendications 1 à 21 pour évaluer la productivité d'une cellule ou colonie de cellules d'hybridome dans la production d'un anticorps intéressant et, facultativement, la spécificité dudit anticorps.

25. Méthode pour sélectionner une cellule ou colonie cellulaire productrice à partir d'une pluralité de cellules ou de colonies cellulaires, méthode comprenant :
(a) l'évaluation de la quantité du polypeptide intéressant produite par une cellule ou colonie cellulaire appropriée par une méthode suivant l'une quelconque des revendications 1 à 21 ;
(b) la comparaison de ladite quantité à une quantité prédéterminée ;
(c) la sélection de la cellule ou colonie cellulaire si elle correspond à ou dépasse la quantité prédéterminée.

26. Méthode suivant la revendication 25 pour sélectionner une série de cellules ou colonies cellulaires productrices correspondant à, ou dépassant, la quantité prédéterminée.

27. Méthode suivant la revendication 25 ou 26 pour détecter une spécificité d'un polypeptide produit par la cellule ou colonie de cellules, qui comprend en outre les étapes consistant :
(a) à évaluer la spécificité de liaison du polypeptide intéressant produit par la cellule ou la colonie de cellules par une méthode suivant l'une quelconque des revendications 1 à 21 ;
(b) à sélectionner la cellule ou colonie de cellules si elle produit un polypeptide qui se lie spécifiquement à un partenaire de liaison intéressant.

28. Méthode suivant l'une quelconque des revendications précédentes, dans laquelle la pluralité de cellules ou colonies de cellules est disposée sur un substrat plan tel qu'une boîte ou plaque de culture.

29. Méthode pour prélever une cellule ou colonie de cellules productrice d'un polypeptide, méthode comprenant l'évaluation de la quantité du polypeptide intéressant produite par la cellule ou colonie de cellules par une méthode suivant l'une quelconque des revendications 20 à 21, et le prélèvement de cette cellule ou colonie de cellules.

30. Méthode suivant l'une quelconque des revendications précédentes, dans laquelle le marqueur de classe se lie au, et agrège le, polypeptide pour permettre la détection d'un signal provenant du marqueur sur le marqueur de spécificité.

31. Utilisation d'un robot équipé d'une tête pour manipuler des échantillons biologiques, utilisation comprenant les étapes consistant :
à fournir un récipient d'échantillon contenant au moins une cellule capable de produire un polypeptide intéressant, le polypeptide ayant été exposé : (a) à un marqueur de classe capable d'identifier une classe de polypeptides à laquelle appartient le polypeptide intéressant ; et (b) à un marqueur de spécificité capable d'identifier une capacité de liaison spécifique du polypeptide intéressant ;
à placer le récipient d'échantillon au niveau d'un poste d'imagerie ;
à effectuer une mesure spectroscopique en éclairant ladite au moins une cellule par le dessous et en collectant la lumière provenant de ladite au moins une cellule également par le dessous afin de détecter la liaison du polypeptide au marqueur de classe ou au marqueur de spécificité ou bien à ces deux marqueurs, dans laquelle le complexe marqueur de classe-polypeptide est associé à, coïncidant avec, ou autour de, la cellule ou colonie de cellules et dans laquelle les dimensions ou l'étendue du complexe sont détectées pour fournir une mesure de la quantité de polypeptide produite par la cellule ou colonie de cellules ; et
à manipuler ladite au moins une cellule avec la tête d'après la mesure spectroscopique de la liaison du polypeptide.

32. Utilisation suivant la revendication 31, dans laquelle la mesure spectroscopique est basée sur l'utilisation d'un agent fluorescent.

33. Utilisation suivant la revendication 32, dans laquelle l'agent fluorescent est un colorant.

34. Utilisation suivant la revendication 33, dans laquelle le colorant est présent dans, sur, ou autour de, ladite au moins une cellule.

35. Utilisation suivant la revendication 33, dans laquelle ladite au moins une cellule est présente dans un milieu qui est marqué avec le colorant dont l'activité optique est modifiée par sécrétion par ladite au moins une cellule.

36. Utilisation suivant la revendication 35, dans laquelle la mesure spectroscopique détecte la fluorescence, la lumière blanche, le rayonnement infrarouge ou l'auto-fluorescence ou bien est effectuée par spectroscopie Raman.

37. Utilisation suivant la revendication 32, dans laquelle l'agent fluorescent fait partie inhérente de la cellule.

38. Utilisation suivant la revendication 31, dans laquelle ladite au moins une cellule est une cellule animale.

39. Utilisation suivant la revendication 32, dans laquelle l'agent fluorescent est détecté par lumière blanche ou rayonnement infrarouge.

40. Utilisation suivant la revendication 31, dans laquelle la mesure spectroscopique est basée sur l'utilisation d'un agent Raman.
